# EUROPEAN PATENT APPLICATION

(11) **EP 4 616 944 A1**
(43) Date of publication of application: **17.09.2025**
(21) Application number: 23887855.7
(22) Date of filing: 31.10.2023
(51) Int. Cl.: B01J 29/70, B01J 29/89

(54) **TI-MWW MOLECULAR SIEVE CATALYST, PREPARATION METHOD THEREFOR, AND USE THEREOF**

(30) Priority: 10.11.2022 CN 202211408458
(71) Applicant: China Petroleum & Chemical Corporation, Beijing 100728 (CN); Sinopec (Shangai) Research Institute of Petrochemical Technology Co., Ltd., Shanghai 201208 (CN)
(72) Inventor: YANG, Weimin, Shanghai 201208 (CN); JIN, Shaoqing, Shanghai 201208 (CN); FAN, Xueyan, Shanghai 201208 (CN); TANG, Zhimou, Shanghai 201208 (CN); SUN, Hongmin, Shanghai 201208 (CN)
(74) Representative: karo IP
(86) International application number: PCT/CN2023/128161
(87) International publication number: WO 2024/099165

(57) **Abstract**

The present disclosure relates to a Ti-MWW molecular sieve catalyst, a preparation method therefor, and the application thereof. An X-ray photoelectron energy spectrum of the catalyst involves peaks at 458.9±0.2 eV and 464.8±0.2 eV, preferably at 458.9±0.2eV 460.3±0.2 eV, 464.8±0.2 eV and 465.9±0.2 eV. When used in epoxidation of olefins, the Ti-MWW molecular sieve catalyst shows advantages such as high conversion for olefins, high selectivity for epoxides, and good catalytic stability.

## Description

### Technical field

The present disclosure relates to the technical field of titanosilicate molecular sieve catalysts, particularly to a Ti-MWW molecular sieve catalyst, its preparation and application.

### Background

Epoxides are important organic chemical materials, mainly including ethylene oxide, propylene oxide, 1-pentene oxide, 1-hexene oxide, and the like. At present, epoxides are basically produced by selective oxidation of olefins. EniChem has developed a method for producing propylene oxide, known as Hydrogen Peroxide to Propylene Oxide (HPPO) (see, for example, US4410501A). It produces propylene oxide via a reaction of propylene and hydrogen peroxide in methanol solvent by using TS-1 titanosilicate molecular sieve having an MFI structure as a catalyst. The process has the advantages of environmental friendliness and high raw material utilization, and has been industrialized. However, due to the fact that TS-1 molecular sieve has 10-membered ring channels which have a size of about 0.5 nm and thereby are disadvantageous for the diffusion of large molecules, it may face great challenges in the epoxidation of cyclohexene which has slightly larger molecular sizes. To solve such challenges, Professor Wu Peng et. al. have developed a new generation of titanosilicate molecular sieve (Ti-MWW) (see Journal of Catalysis, 2001, 202, 245). Compared with a TS-1 molecular sieve, a Ti-MWW molecular sieve has not only higher conversion for olefins, but also better selectivity for epoxides.

The current industrial production of propylene oxide is mainly operated in fixed bed reactors. The titanosilicate molecular sieves obtained by hydrothermal method are powders with a size of microns or nanometers having no mechanical strength. When being directly loaded into the fixed bed reactors, they may be entrained into pipelines of the device by the reaction liquids during the reaction, resulting in pipeline blockage. In addition, it is very difficult to separate and recover the titanosilicate molecular sieve powders from the reaction liquids after the reaction. In order to ensure efficient and continuous operation of the reaction in fixed beds, titanosilicate molecular sieve powders need be made into a catalyst with good mechanical strength.

CN1346705A proposes to use small balls with certain mechanical strength as supports, and to enrich titanosilicate molecular sieves on surface of the balls by shaping via spheronizing. It improves the mechanical strength of the obtained catalyst. CN112354557A discloses preparation and application of an integral titanozeolite catalyst. An integral catalyst for continuously epoxidizing propylene is prepared by mixing an amorphous silicon-based binder and a polymeric pore-forming agent into an MWW titanium zeolite powder, adding water, stirring and kneading, mechanically shaping and calcining, immersing into an aqueous solution of a cyclic nitrogen-containing organic composite, sealing, heating, filtering, drying and calcining.

On the whole, Ti-MWW molecular sieve catalysts of the prior art, whether or not contain binders, suffer from low mechanical strength and poor catalytic performance. Theoretically, the composition and structure of Ti-MWW molecular sieve catalysts of the prior art can be modified to further improve their mechanical strength and catalytic performance. Therefore, there is always a demand in the art for developing Ti-MWW molecular sieve catalysts with high mechanical strength and good catalytic performance.

### Summary of the invention

The present disclosure is to address the problems in the prior art, such as, the low mechanical strength and poor catalytic performance of Ti-MWW molecular sieve catalysts. Provided in the present disclosure is a Ti-MWW molecular sieve catalyst, a method for preparing the same and use of the same. The Ti-MWW molecular sieve catalyst in accordance with the present disclosure comprises titanium species in a good state, preferably is in a fully crystalline structure, thereby showing advantages of high mechanical strength and excellent catalytic performance.

To achieve the above-mentioned purpose, in the first aspect, provided in the present disclosure is a Ti-MWW molecular sieve catalyst, wherein an X-ray photoelectron energy spectrum of the catalyst involves peaks at 458.9±0.2 eV and 464.8±0.2 eV, preferably at 458.9±0.1 eV and 464.8±0.1 eV; preferably, the X-ray photoelectron energy spectrum of the catalyst involves peaks at 458.9±0.2 eV, 460.3±0.2 eV, 464.8±0.2 eV and 465.9±0.2 eV, preferably at 458.9±0.1 eV, 460.3±0.1 eV, 464.8±0.1 eV and 465.9±0.1 eV.

In the second aspect, provided in the present disclosure is a method for preparing a Ti-MWW molecular sieve catalyst, comprising steps of:
(1) subjecting a Ti-MWW molecular sieve powder, a binder, a pore-forming agent and a fluoride to shaping and calcining, to obtain a shaped product;
(2) crystallizing the shaped product of step (1) in the presence of an organic amine solution, to obtain a catalyst precursor A;
(3) treating the catalyst precursor A of step (2) with an acid solution, and calcining, to obtain a catalyst precursor B;
(4) treating the catalyst precursor B of step (3) with an organic amine solution, to obtain the catalyst.

At the same time, provided in the present disclosure is a Ti-MWW molecular sieve catalyst prepared by the above method.

In addition, provided in the present disclosure is use of the Ti-MWW molecular sieve catalyst in epoxidation of olefins.

As examples, the present disclosure may include the following items.
1. A fully crystalline Ti-MWW molecular sieve catalyst, whose UV Raman spectrum involves peaks at 343±4 cm⁻¹, 484±4 cm⁻¹, 699±4 cm⁻¹ and 1097±4 cm⁻¹, wherein intensity of the peak at 699±4 cm⁻¹ is 0.5-10 times, preferably 2-10 times that of the peak at 343±4 cm⁻¹, and wherein intensity of the peak at 1097±4 cm⁻¹ is 0.5-10 times, preferably 2-10 times that of the peak at 343±4 cm⁻¹.
2. The molecular sieve catalyst according to item 1, characterized in that, a molar ratio of silicon to titanium of the molecular sieve catalyst is 10-200, preferably 25-100; the molecular sieve catalyst further comprises at least one element of boron and aluminum, preferably boron; a molar ratio of boron to silicon of the molecular sieve catalyst is 0-0.1, preferably 0-0.03, more preferably 0.005-0.03; a molar ratio of aluminum to silicon of the molecular sieve catalyst is 0-0.1, preferably 0-0.05.
3. The molecular sieve catalyst according to item 1, characterized in that, the molecular sieve catalyst has a volume of micropores of 0.03-0.15 cm³/g, preferably 0.03-0.12 cm³/g, more preferably 0.05-0.10 cm³/g; wherein propotion of the volume of micropores to total volume of pores is 1%-7.5%, preferably 1-6%, more preferably 1.7%-5%.
4. The molecular sieve catalyst according to item 1, characterized in that, the molecular sieve catalyst has a mechanical strength of 30-90 N/cm, preferably 40-80 N/cm.
5. A method for preparing a fully crystalline Ti-MWW molecular sieve catalyst, comprising steps of:
   (1) subjecting a Ti-MWW molecular sieve powder, a binder, a pore-forming agent and a fluoride to kneading, shaping and calcining, to obtain a shaped product;
   (2) crystallizing the shaped product of step (1) in an environment of organic amine solution, to obtain a catalyst precursor A;
   (3) treating the catalyst precursor A of step (2) with an acid solution, and calcining, to obtain a catalyst precursor B;
   (4) treating the catalyst precursor B of step (3) with an organic amine solution, to obtain the molecular sieve catalyst.
6. The method according to item 5, characterized in that, the binder in step (1) comprises a silicon source and at least one selected from the group consisting of a boron source and an aluminum source; wherein the binder comprises ingredients such that, on oxides basis, SiO₂, B₂O₃ and Al₂O₃ are in a molar ratio of 1:x:y, where x=0-0.5, y=0-0.5, and x+y=0.02-1.
7. The method according to item 6, characterized in that, the silicon source is at least one selected from the group consisting of silica sol, sodium silicate, white carbon black and ethyl orthosilicate; the boron source is at least one selected from the group consisting of boric acid, boron trioxide and borates; the aluminum source is at least one selected from the group consisting of aluminum oxide, aluminum hydroxide, sodium metaaluminate, aluminum nitrate and aluminum sulfate.
8. The method according to any one of items 5-7, characterized in that, the pore-forming agent in step (1) is at least one selected from the group consisting of sesbania powder, cellulose, chitosan, lignin, starch, polyethylene glycol, triblock copolymers P123 and F127; the fluoride in step (1) is at least one selected from the group consisting of sodium fluoride, potassium fluoride and ammonium fluoride; among raw materials in step (1), the Ti-MWW molecular sieve powder, the binder, the pore-forming agent and the fluoride are in a mass ratio of 1:0.1-1.5:0.01-0.1:0.01-0.4.
9. The method according to item 5, characterized in that, step (2) crystallizing in the environment of organic amine solution comprises: subjecting the shaped product of step (1) to crystallizing by placing the same over the organic amine solution, wherein the shaped product is not in contact with the organic amine solution; wherein the organic amine is at least one selected from the group consisting of piperidine and hexamethyleneimine; the organic amine solution has a concentration of 0.3-15 mol/L; the shaped product and organic amine solution are in a mass ratio of 0.1-10:1; and the crystallizing is operated under conditions of: at a temperature of 130-190°C for 1-9 days.
10. The method according to item 5, characterized in that, step (3) treating with the acid solution comprises: subjecting the catalyst precursor A of step (2) and the acid solution to contacting and reacting; wherein the acid solution is at least one selected from the group consisting of solutions of nitric acid, hydrochloric acid, sulfuric acid, formic acid, acetic acid and oxalic acid; the acid solution has a concentration of 0.3-12 mol/L; the catalyst precursor A and the acid solution are in a solid-liquid ratio by mass of 1:10-80; the treating with the acid solution is operated under conditions of: at a temperature of 60-130°C for 4-48 hours.
11. The method according to item 5, characterized in that, step (4) treating with the organic amine solution comprises: subjecting the catalyst precursor B of step (3), the fluoride and the organic amine solution to contacting and reacting; wherein the fluoride is at least one selected from the group consisting of sodium fluoride, potassium fluoride and ammonium fluoride; the organic amine is at least one selected from the group consisting of piperidine and hexamethyleneimine; the organic amine solution has a concentration of 0.3-15 mol/L; the catalyst precursor B, the fluoride and the organic amine solution are in a mass ratio of 1:0.05-0.4:2-20; the treating with the organic amine solution is operated under conditions of: at a temperature of 130-190°C for 4-48 hours.
12. The method according to item 5, characterized in that, the calcining in step (1) is operated under conditions of: at 450-650°C under an oxygen-containing atmosphere for 4-12 hours; the calcining in step (3) is operated under conditions of: at 450-650°C under an oxygen-containing atmosphere for 4-12 hours.
13. A molecular sieve catalyst prepared by the method according to any one of items 5-12.
14. Use of the molecular sieve catalysts according to any one of items 1-4 or the molecular sieve catalysts according to item 13 in epoxidation of olefins.

Compared with the prior art, the present invention has the following advantages.
1. The Ti-MWW molecular sieve catalyst in accordance with the present disclosure involves a large amount of titanium species in good state, including modified extra-framework hexacoordinated titanium species, or including framework tetracoordinated titanium species and modified extra-framework hexacoordinated titanium species. Those titanium species in good state impart the catalyst with higher catalytic performance. Moreover, the Ti-MWW molecular sieve catalyst preferably is in a fully crystalline structure. It means that the catalyst contains no amorphous binder. In other words, the binder does not exist in an amorphous form, but is converted into an MWW molecular sieve, and then becomes a part of the final catalyst, thereby making the obtained catalyst in a fully crystalline structure. On the one hand, the absence of an amorphous binder means that the effects of the amorphous binder on shielding the catalytic active center and blocking pores of the molecular sieve are avoided, thereby improving the activity and stability of the catalyst. On the other hand, the fully crystalline structure means that the mechanical strength is higher and the catalyst is not prone to pulverization and loss. When used in epoxidation of olefins, the catalyst shows advantages of high conversion for olefins, high selectivity for epoxides, and good catalytic stability.
2. In the preparation in accordance with the present disclosure, the preferred amorphous binder includes a silicon source and a boron source, which can be efficiently converted into an MWW molecular sieve, thereby making the obtained catalyst in a fully crystalline structure, and then improving the catalytic performance and mechanical strength. In addition, the preparation treats the crystallized catalyst precursor A with an acid solution and an organic amine solution, respectively, which effectively converts titanium species in the Ti-MWW molecular sieve into titanium species in good state, including a modified extra-framework hexacoordinated titanium species and optionally a framework tetracoordinated titanium species. The preparation in accordance with the present disclosure obtains the Ti-MWW molecular sieve catalyst with excellent catalytic activity, selectivity and stability.
3. When used in epoxidation of olefins, the catalyst in accordance with the present disclosure shows excellent catalytic performance, high conversion for olefins, high selectivity for epoxides, good catalytic stability, and thereby has good application prospects.

### Description of the drawings

Fig. 1 is an X-ray photoelectron spectrum of the Ti-MWW molecular sieve catalyst obtained in Example 1;
Fig. 2 is a UV Raman spectrum of the Ti-MWW molecular sieve catalyst obtained in Example 1;
Fig. 3 is an X-ray diffraction pattern of the Ti-MWW molecular sieve catalyst obtained in Example 1;
Fig. 4 is a scanning electron microscope image of the Ti-MWW molecular sieve catalyst obtained in Example 1;
Fig. 5 is an X-ray photoelectron spectrum of the Ti-MWW molecular sieve catalyst obtained in Comparative Example 1;
Fig. 6 is a UV Raman spectrum of the Ti-MWW molecular sieve catalyst obtained in Comparative Example 1;
Fig. 7 is an X-ray diffraction pattern of the Ti-MWW molecular sieve catalyst obtained in Comparative Example 1;
Fig. 8 is a scanning electron microscope image of the Ti-MWW molecular sieve catalyst obtained in Comparative Example 1;
Fig. 9 is a UV Raman spectrum of the Ti-MWW molecular sieve catalyst obtained in Comparative Example 2;
Fig. 10 is a UV Raman spectrum of the Ti-MWW molecular sieve catalyst obtained in Comparative Example 3;
Fig. 11 is a UV Raman spectrum of the Ti-MWW molecular sieve catalyst obtained in Comparative Example 4;
Fig. 12 is a UV Raman spectrum of the Ti-MWW molecular sieve catalyst obtained in Comparative Example 5;
Fig. 13 is an X-ray photoelectron spectrum of the Ti-MWW molecular sieve catalyst obtained in Comparative Example 6;
Fig. 14 is a UV Raman spectrum of the Ti-MWW molecular sieve catalyst obtained in Comparative Example 6;
Fig. 15 is an X-ray photoelectron spectrum of the Ti-MWW molecular sieve catalyst obtained in Comparative Example 7;
Fig. 16 is a UV Raman spectrum of the Ti-MWW molecular sieve catalyst obtained in Comparative Example 7.

### Detailed description

In the present invention, unless otherwise stated, percentages and proportions are by mass. Unless otherwise stated, throughout the specification and claims, the term "comprise" or its variations such as "comprises" or "comprising" and the like shall be understood to include the stated steps or components, without excluding other steps or other components.

Other than in the examples, all numerical values of parameters in this specification are to be understood as being modified in all instances by the term "about" whether or not "about" actually appears before the numerical value.

In one embodiment, provided in the present disclosure is a Ti-MWW molecular sieve catalyst, wherein an X-ray photoelectron energy spectrum of the catalyst involves peaks at 458.9±0.2 eV and 464.8±0.2 eV, preferably at 458.9±0.1 eV and 464.8±0.1 eV; preferably, the X-ray photoelectron energy spectrum of the catalyst involves peaks at 458.9±0.2 eV, 460.3±0.2 eV, 464.8±0.2 eV, and 465.9±0.2 eV, preferably at 458.9±0.1 eV, 460.3±0.1 eV, 464.8±0.1 eV, and 465.9±0.1 eV. The peaks at 460.3±0.2 eV and 465.9±0.2 eV are attributed to the framework tetracoordinated titanium species; the peaks at 458.9±0.2 eV and 464.8±0.2 eV are attributed to the modified extra-framework hexacoordinated titanium species.

As used herein, the expression "Ti-MWW molecular sieve" refers to a titanosilicate molecular sieve with a three-dimensional MWW structure. Ti-MWW molecular sieves are commercially available or can be prepared according to known methods in the art. Generally, Ti-MWW molecular sieves are synthesized by a hydrothermal method using boric acid as a crystallization aid (see, for example, Journal of Physical Chemistry B, 2001, 105, 2897). Ti-MWW molecular sieves prepared by a hydrothermal method are referred to herein as "as-synthesized Ti-MWW molecular sieve powder". Methods are proposed in the art for modifying as-synthesized Ti-MWW molecular sieve powders to improve their catalytic performance. For example, the as-synthesized Ti-MWW molecular sieve powder can be acid-treated (see, for example, Journal of Catalysis, 2001, 202, 245). The as-synthesized Ti-MWW molecular sieve powder after the acid-treating is referred to herein as "Ti-MWW molecular sieve powder".

Titanium species in a Ti-MWW molecular sieve may be in the forms of framework tetracoordinate, extra-framework hexacoordinate and titanium dioxide. The as-synthesized Ti-MWW molecular sieve powder prepared by a hydrothermal method may contain a small amount of framework tetracoordinate titanium species and a large amount of extra-framework hexacoordinate titanium species. The framework tetracoordinate titanium species are generally regarded as catalytic active centers for epoxidation of olefins, i.e. so-called "titanium species in good state". However, the extra-framework hexacoordinate titanium species and titanium dioxide are not the catalytic active centers for epoxidation of olefins, i.e. so-called "titanium species in bad state". The prior art makes effort to convert extra-framework hexacoordinate titanium species into framework tetracoordinate titanium species, so as to improve the catalytic performance of the catalyst. The above-mentioned acid-treating of the as-synthesized Ti-MWW molecular sieve powder is just such conversion.

It is surprisingly found by the inventor that, extra-framework hexacoordinated titanium species or framework tetracoordinated titanium species can be converted into modified extra-framework hexacoordinated titanium species. The modification of titanium species can be reflected by peaks in X-ray photoelectron spectrums of Ti-MWW molecular sieves. Before the modification, the X-ray photoelectron spectrum of the as-synthesized Ti-MWW molecular sieve powder may have peaks at 458.0±0.2 eV and 463.8±0.2 eV, which are attributed to extra-framework hexacoordinated titanium species. Alternatively, the X-ray photoelectron spectrum of the Ti-MWW molecular sieve powder may have peaks at 460.3±0.2 eV and 465.9±0.2 eV, which are attributed to framework tetracoordinated titanium species. After the modification, the X-ray photoelectron spectrum of the Ti-MWW molecular sieve powder may have peaks at 458.9±0.2 eV and 464.8±0.2 eV, which are attributed to modified extra-framework hexacoordinated titanium species. The modified extra-framework hexacoordinated titanium species also has excellent catalytic activity on epoxidation of olefins. That is, it is also a titanium species in good state, thereby can impart improved catalytic performance to the Ti-MWW molecular sieve catalyst in accordance with the present disclosure. The present invention was completed based on the above findings.

Preferably, a UV Raman spectrum of the catalyst involves peaks at 343±4 cm⁻¹, 484±4 cm⁻¹, 699±4 cm⁻¹ and 1097±4 cm⁻¹, wherein intensity of the peak at 699±4 cm⁻¹ is 0.5-10 times, preferably 2-10 times that of the peak at 343±4 cm⁻¹, and wherein intensity of the peak at 1097±4 cm⁻¹ is 0.5-10 times, preferably 2-10 times that of the peak at 343±4 cm⁻¹. Generally, the peak at 343±4 cm⁻¹ is attributed to MWW framework, the peaks at 484±4 cm⁻¹ and 1097±4 cm⁻¹ are attributed to the framework tetracoordinated titanium species, and the peak at 699±4 cm⁻¹ is attributed to the extra-framework hexacoordinated titanium species and/or modified extra-framework hexacoordinated titanium species.

Preferably, a molar ratio of silicon to titanium of the catalyst (n_{Si}/n_{Ti}) is 10-200, preferably 25-100, on atom basis.

Preferably, the catalyst may further comprise at least one element of boron and aluminum, preferably boron. A molar ratio of boron to silicon (n_{B}/n_{Si}) is 0-0.1, preferably 0-0.03, more preferably 0.005-0.03, on atom basis. A molar ratio of aluminum to silicon (n_{Al}/n_{Si}) is 0-0.1, preferably 0-0.03, on atom basis.

Preferably, the catalyst is in a porous structure, and includes micropores, mesopores and macropores. The micropores may have a pore size of less than 2 nm, such as 0.4-2 nm; the mesopores may have a pore size of 2-50 nm; the macropores may have a pore size of greater than 50 nm, such as 50-500 nm. In one variation, the catalyst has a volume of micropores of 0.03-0.15 cm³/g, preferably 0.03-0.12 cm³/g, more preferably 0.05-0.10 cm³/g. A propotion of the volume of micropores to total volume of pores is 1-7.5%, preferably 1-6%, more preferably 1.7-5%.

Preferably, the catalyst is in a fully crystalline structure. As used herein, the expression "fully crystalline" refers to the fact that the molecular sieve catalyst is free or substantially free of an amorphous binder. "Substantially free" refers to the fact that the catalyst comprises the amorphous binder in an amount of less than 5 weight %, preferably less than 3 weight %, more preferably less than 1 weight %. The binder is converted into an MWW molecular sieve, thereby becoming a part of the resulting catalyst. The fully crystalline structure can be determined by scanning electron microscopy and X-ray diffraction.

Preferably, the catalyst has a mechanical strength of 30-90 N/cm, preferably 40-80 N/cm.

In a further embodiment, provided in the present disclosure is a method for preparing a Ti-MWW molecular sieve catalyst, comprising steps of:
(1) subjecting a Ti-MWW molecular sieve powder, a binder, a pore-forming agent and a fluoride to shaping and calcining, to obtain a shaped product;
(2) crystallizing the shaped product of step (1) in the presence of an organic amine solution, to obtain a catalyst precursor A;
(3) treating the catalyst precursor A of step (2) with an acid solution, and calcining, to obtain a catalyst precursor B;
(4) treating the catalyst precursor B of step (3) with an organic amine solution, to obtain the catalyst.

Preferably, a molar ratio of silicon to titanium of the Ti-MWW molecular sieve powder is 5-120, on atom basis. The Ti-MWW molecular sieve powder is commercially available or can be prepared according to the techniques disclosed in the art.

Preferably, the binder is an amorphous binder and comprises a silicon source and at least one selected from the group consisting of a boron source and an aluminum source. Preferably, the amorphous binder comprises a silicon source and a boron source. More preferably, on oxides basis, the silicon source, the boron source and the aluminum source are in a molar ratio of 1:x:y, where x=0-0.5, y=0-0.5, and x+y=0.02-1. Preferably, the silicon source is at least one selected from the group consisting of silica sol, sodium silicate, white carbon black and ethyl orthosilicate; the boron source is at least one selected from the group consisting of boric acid, boron trioxide and borates; the aluminum source is at least one selected from the group consisting of aluminum oxide, aluminum hydroxide, sodium metaaluminate, aluminum nitrate and aluminum sulfate. The binder is commercially available or can be prepared according to the techniques disclosed in the art. In one variation, the binder is prepared by mixing the components (e.g. the silicon source, the boron source, and the aluminum source).

Preferably, the pore-forming agent is at least one selected from the group consisting of sesbania powder, cellulose, chitosan, lignin, starch, polyethylene glycol, a triblock copolymer of poly(ethylene oxide)-poly(propylene oxide)-poly(ethylene oxide) (P123) and a copolymer of poly(ethylene oxide)-poly(propylene oxide) (F127).

Preferably, the fluoride is at least one selected from the group consisting of sodium fluoride, potassium fluoride and ammonium fluoride.

Preferably, in step (1), the Ti-MWW molecular sieve powder, the binder, the pore-forming agent and the fluoride are used in a mass ratio of 1:(0.1-1.5):(0.01-0.1):(0.01-0.4).

Preferably, in step (1), before shaping, the Ti-MWW molecular sieve powder, the binder, the pore-forming agent and the fluoride are subjecting to kneading, preferably in the presence of water. Optionally, in step (1), after kneading and shaping, the product is subjecting to drying. Preferably, the drying is operated at 60-120 °C for 1-24 hours.

Preferably, in step (1), the calcining is operated at 450-650 °C under an oxygen-containing atmosphere for 4-12 hours. The oxygen-containing atmosphere may be air or oxygen, and preferably air.

Preferably, in step (2), the shaped product of step (1) is placed over the organic amine solution but not in contact with the organic amine solution.

Preferably, in step (2), the organic amine is at least one selected from the group consisting of piperidine and hexamethyleneimine. In one variation, the organic amine solution has a concentration of 0.3-15 mol/L. Preferably, the shaped product and organic amine solution are used in a mass ratio of (0.1-10):1. In one variation, the crystallizing is operated in a closed environment at a temperature of 130-190 °C and the autogenous pressure for 1-9 days.

Preferably, step (2) further comprises: after crystalizing, subjecting the product to washing and drying. Preferably, the washing is washing by water. Preferably, the drying is operated at 60-120 °C for 1-24 hours.

Preferably, step (2) does not comprise calcining.

Preferably, step (3) comprises: subjecting the catalyst precursor A of step (2) and the acid solution to contacting and reacting. The acid solution is at least one selected from the group consisting of solutions of nitric acid, hydrochloric acid, sulfuric acid, formic acid, acetic acid and oxalic acid. The acid solution has a concentration of 0.3-12 mol/L. The catalyst precursor A and the acid solution are used in a mass ratio of 1:(10-80). The treating with the acid solution is operated at a temperature of 60-130°C for 4-48 hours.

Optionally, in step (3), after treating with the acid solution, the product is subjecting to washing and drying. Preferably, the washing is washing by water. Preferably, the drying is operated at 60-120 °C for 1-24 hours.

Preferably, in step (3), the calcining is operated at 450-650 °C under an oxygen-containing atmosphere for 4-12 hours. The oxygen-containing atmosphere may be air or oxygen, and preferably air.

Preferably, step (4) comprises: subjecting the catalyst precursor B of step (3) and the organic amine solution to contacting and reacting in the presence of the fluoride. The fluoride is at least one selected from the group consisting of sodium fluoride, potassium fluoride and ammonium fluoride. The organic amine is at least one selected from the group consisting of piperidine and hexamethyleneimine. The organic amine solution has a concentration of 0.3-15 mol/L. The catalyst precursor B, the fluoride and the organic amine solution are used in a mass ratio of 1:(0.05-0.4):(2-20). The treating with the organic amine solution is operated at a temperature of 130-190°C for 4-48 hours.

Optionally, in step (4), after treating with the organic amine solution, the product is subjecting to washing and drying. Preferably, the washing is washing by water. Preferably, the drying is operated at 60-120 °C for 1-24 hours.

Preferably, step (4) does not comprise calcining.

In a further embodiment, provided in the present disclosure is a Ti-MWW molecular sieve catalyst prepared by the above method. The Ti-MWW molecular sieve catalyst has all characteristics of the Ti-MWW molecular sieve catalyst in accordance with the present disclosure as described above, which is not repeated here.

In a further embodiment, provided in the present disclosure is use of the Ti-MWW molecular sieve catalyst in epoxidation of olefins.

Preferably, the use comprises steps of: mixing olefins, aqueous hydrogen peroxide solution, solvents and alkaline nitrogen-containing materials, to form a feed solution, and subjecting the feed solution and the catalyst to contacting and reacting. In one variation, the reaction is operated in a fixed bed reactor.

Preferably, the olefins are liquefied olefins. The olefins include at least one selected from the group consisting of propylene, allyl chloride, butene, pentene, cyclopentene, hexene and cyclohexene. The aqueous hydrogen peroxide solution has a concentration of 10-70% by mass. The solvents are at least one selected from the group consisting of methanol, acetonitrile, propionitrile, acetone and tert-butyl alcohol. The alkaline nitrogen-containing materials are at least one selected from the group consisting of piperidine and hexamethyleneimine.

Preferably, in the feed solution, the olefins and the hydrogen peroxide are in a molar ratio of 1:0.3-1. The olefins are present in the feed solution in an amount of 1-50% by mass; the solvents are present in the feed solution in an amount of 30-90% by mass; and the alkaline nitrogen-containing materials are present in the feed solution in an amount of 1-50 ppm.

Preferably, the reaction is operated under the conditions of: at a flow rate of the catalyst per unit mass of the feed solution of 3-30 mL·g_{cat.}⁻¹·h⁻¹, at a temperature of 30-100 °C and a pressure of 0.1-4 MPa.

### Examples

The features and advantages of the invention will become apparent from the following examples. The examples are intended to illustrate and not to limit the invention in any way.

### Methods for testing

In the present disclosure, for example, in the following examples and comparative examples, the type, state, structure and morphology of titanium species of the molecular sieve catalyst were determined respectively by UV Raman spectroscopy, X-ray photoelectron spectroscopy, X-ray diffraction, and scanning electron microscopy. The molar ratio of silicon to titanium, the molar ratio of boron to silicon, and the molar ratio of aluminum to silicon of the molecular sieve catalyst were determined by inductively coupled atomic emission spectroscopy. The volume of micropores, the volume of mesopores, and the volume of macropores of the molecular sieve catalyst were determined respectively by nitrogen adsorption and desorption and mercury intrusion porosimetry. The results were used to calculate the proportion of the volume of micropores to the total volume of pores as follow. That is, the proportion of the volume of micropores to the total volume of pores = the volume of micropores divided by the sum of the volume of micropores, the volume of mesopores, and the volume of macropores. The mechanical strength of the molecular sieve catalyst was determined by a strength tester.

In the present disclosure, the testing via UV Raman spectroscopy comprised: operating the testing on a domestic UV Raman-100 UV Raman spectrometer, with an excitation wavelength of 244 nm, a laser power of 5.0 mW on the sample, and a spectral resolution of 4 cm⁻¹, to obtain the UV Raman spectrum of the molecular sieve catalyst. The intensity of a peak is obtained by subtracting the value of the baseline from the value of the peak.

In the present disclosure, the testing via X-ray photoelectron spectroscopy comprised: operating the testing on AXIS Ultra DLD X-ray photoelectron spectrometer, with Al Kα as the ray source (1486.6 eV), the sample chamber being vacuumized to a pressure of 10⁻⁹ Torr, and calibrating with a peak of C 1s at 284.8 eV. OriginPro 9 was used for peak fitting.

In the present disclosure, the testing via X-ray diffraction comprised: analyzing the sample on Rigaku UlTima IV X-ray powder diffractometer, with Cu Kα as the ray source (λ = 1.54 Å), a nickel filter, 2θ scanning range of 2-50°, an operating voltage of 40 kV, a current of 40 mA, a scanning rate of 10°/min.

In the present disclosure, the testing via scanning electron microscope comprised: operating the testing on Hitachi S-4800 electron microscope with an acceleration voltage of 3 kV.

In the present disclosure, the testing via inductively coupled atomic emission spectrometry comprised: analyzing the sample on Varian-2000 analyzer for the molar ratio of silicon-titanium, the molar ratio of boron-silicon, and the molar ratio of aluminum-silicon of the sample, wherein the sample was dissolved in a hydrofluoric acid solution before the testing.

In the present disclosure, the testing via nitrogen adsorption-desorption comprised: determining the nitrogen adsorption-desorption isotherm of the sample on American Micromeritics ASAP2460 instrument, on which the volume of micropores was obtained, wherein the determining was operated at a temperature of 77 K, and the sample was vacuum pretreated at 573 K for 6 hours before the testing.

In the present disclosure, the testing via mercury intrusion porosimetry comprised: operating the testing on a high-performance fully automatic mercury intrusion instrument AutoPore IV 9505.

In the present disclosure, the testing for mechanical strength comprised: operating the testing on a DL-2 particle strength tester. In particular, the size of the catalyst in the direction of the applied force was determined, and then the external force required to crush the catalyst into powder was determined. The mechanical strength of the catalyst was obtained by dividing the external force by the size.

In the present disclosure, for example, in the following examples and comparative examples, the catalytic performance of the molecular sieve catalyst was characterized by the propotion of residual hydrogen peroxide, the conversion of hydrogen peroxide, the selectivity for the main products (such as epoxides) and the selectivity for the by-products (such as glycols and alcohol ethers), the ratio of the main products to the by-products, and the period for keeping stability of the catalyst.

In the present disclosure, a sample of the feed solution was obtained from an inlet of the reaction tube, and a sample of the reaction liquid was obtained from an outlet of the reaction tube. The sample of the reaction liquid could be obtained at any stage of the reaction, for example, when the reaction liquid was just emerged from the outlet of the reaction tube, during the reaction, or at the end of the reaction. The sample of the feed solution and the sample of the reaction liquid were tested for the concentration of hydrogen peroxide therein by titrating with cerium sulfate. The results were used for calculating the propotion of residual hydrogen peroxide and the conversion of hydrogen peroxide according to the following formulas. the propotion of residual hydrogen peroxide (%) = the concentration of hydrogen peroxide in the reaction liquid (mol/L) / the concentration of hydrogen peroxide in the feed solution (mol/L) * 100% the conversion of hydrogen peroxide (%) = 1- the propotion of residual hydrogen peroxide (%)

In the present disclosure, the sample of the feed solution and the sample of the reaction liquid were analyzed via gas chromatography for their composition. The results were used for determining the amount of olefins converted in the reaction, the amount of epoxide products generated in the reaction, and thereby calculating the selectivity for the main products (i.e. epoxides) and the ratio of the main products (i.e. epoxides) to the by-products (i.e. glycols and alcohol ethers). the molar amount of olefins converted in the reaction = the molar amount of olefins in the sample of the feed solution - the molar amount of olefins in the sample of the reaction liquid the selectivity for epoxides (%) = the molar amount of epoxide products in the sample of the reaction liquid / the molar amount of olefins converted in the reaction * 100% the ratio of the main products to the by-products = the molar amount of epoxide products in the sample of the reaction liquid / (the molar amount of olefins converted in the reaction - the molar amount of epoxide products in the the sample of reaction liquid) * 100%

In the present disclosure, the period for keeping stability of the catalyst was the time started from the begining of the reaction and ended when the propotion of residual hydrogen peroxide in the sample of the reaction liquid was 2%.

In the examples and comparative examples, the Ti-MWW molecular sieve powder was RT-03B commercially available from Zhejiang TWRD New Materials Co., Ltd., wherein titanium species thereof were only framework tetracoordinated titanium species. The as-synthesized Ti-MWW molecular sieve powder was prepared according to the reference (Journal of Physical Chemistry B, 2001, 105, 2897), which contained only extra-framework hexacoordinated titanium species.

### Example 1

(1) 90 g of Ti-MWW molecular sieve powder having a molar ratio of silicon-titanium of 20, 90 g of an amorphous binder (the amorphous binder comprised 75 g of a silica sol containing silica in a mass fraction of 40% and 15 g of boric acid), 3 g of sesbania powder and 9 g of sodium fluoride were mixed under mechanical stirring, to which 80 g of water was added. After stirring and kneading for 4 hours, obtained was a solid mixture, which was subjected to shaping by mechanical extruding, drying at 100 °C for 8 hours, and calcining at 550 °C for 6 hours, to obtain a cylindrical shaped product.
(2) 90 g of the cylindrical shaped product of step (1) was placed over 60 g of a 3 mol/L piperidine solution without contacting the same, to be subjected to crystallizing at 170 °C for 2 days in a closed environment. The product was subjected to washing with water and drying at 100 °C for 8 hours, to obtain a cylindrical catalyst precursor A.
(3) 60 g of the cylindrical catalyst precursor A of step (2) and 2 mol/L nitric acid solution were subjected to mixing at a mass ratio of 1:50, reacting at 80°C for 24 hours, washing with water, drying at 100°C for 8 hours and calcining at 550°C for 6 hours, to obtain a cylindrical catalyst precursor B.
(4) 40 g of the cylindrical catalyst precursor B of step (3), ammonium fluoride and 3 mol/L piperidine solution were subjected to mixing at a mass ratio of 1:0.1:10, and reacting at 170°C for 24 hours. The mixture was subjected to washing with water and drying at 100°C for 8 hours, to obtain a Ti-MWW molecular sieve catalyst, which was recorded as S1.

The catalyst S1 was subjected to testings as described above.

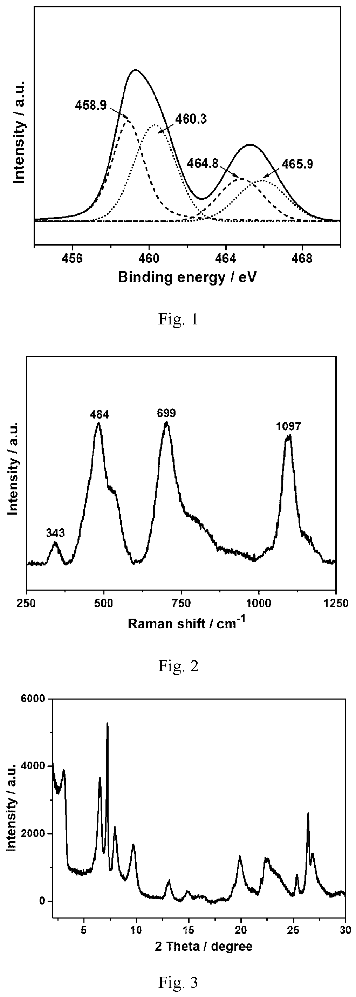

An X-ray photoelectron spectrum of the catalyst S1 was shown in Fig. 1, which showed peaks at 458.9, 460.3, 464.8 and 465.9 eV. Among them, the peaks at 460.3 and 465.9 eV were attributed to framework tetracoordinated titanium species, and the peaks at 458.9 and 464.8 eV were attributed to the modified extra-framework hexacoordinated titanium species.

A UV Raman spectrum of the catalyst S1 was shown in Fig. 2, which showed peaks at 343, 484, 699 and 1097 cm⁻¹. The intensity of the peak at 699 cm⁻¹ was 5.3 times that of the peak at 343 cm⁻¹, and the intensity of the peak at 1097 cm⁻¹ was 5.1 times that of the peak at 343 cm⁻¹. Among them, the peak at 343 cm⁻¹ was attributed to the MWW structure, the peaks at 484 and 1097 cm⁻¹ were attributed to framework tetracoordinated titanium species, and the peak at 699 cm⁻¹ was attributed to the modified extra-framework hexacoordinated titanium species.

An X-ray diffraction pattern of the catalyst S1 was shown in Fig. 3. There were strong diffraction peaks at 2θ of 3.3°, 6.6°, 7.2°, 7.9°, 9.7° and 26.1°, and the intensity of the diffraction peak at 2θ of 7.2° reached 5300, indicating that S1 had an MWW structure and high crystallinity.

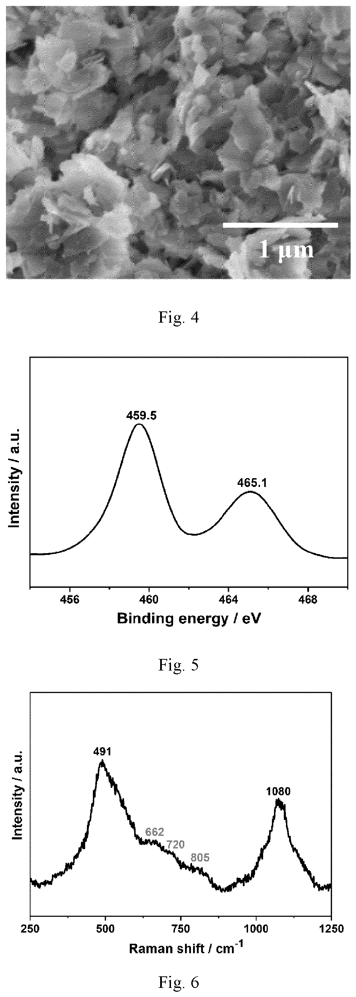

A scanning electron microscope image of the catalyst S1 was shown in Fig. 4. The catalyst S1 showed a lamellar morphology, with no nanoparticles being observed, which indicated that S1 was only in MWW structure.

The catalyst S1 had a molar ratio of silicon-titanium of 35 and a molar ratio of boron-silicon of 0.015.

The catalyst S1 had a volume of micropores of 0.09 cm³/g, a proportion of the volume of micropores to the total volume of pores of 3.6%, and a mechanical strength of 66 N/cm.

### Example 2

(1) 90 g of Ti-MWW molecular sieve powder having a molar ratio of silicon-titanium of 5, 90 g of an amorphous binder (the amorphous binder comprised 75 g of a silica sol containing silica in a mass fraction of 40% and 15 g of boric acid), 3 g starch and 0.9 g potassium fluoride were mixed under mechanical stirring, to which 80 g of water was added. After stirring and kneading for 4 hours, obtained was a solid mixture, which was subjected to shaping by mechanical extruding, drying at 100 °C for 8 hours, and calcining at 550 °C for 6 hours, to obtain a cylindrical shaped product.
(2) 90 g of the cylindrical shaped product of step (1) was placed over 60 g of a 3 mol/L piperidine solution without contacting the same, to be subjected to crystallizing at 170 °C for 2 days in a closed environment. The product was subjected to washing with water and drying at 100 °C for 8 hours, to obtain a cylindrical catalyst precursor A.
(3) 60 g of the cylindrical catalyst precursor A of step (2) and 2 mol/L nitric acid solution were subjected to mixing at a solid-liquid ratio by mass of 1:50, reacting at 80°C for 24 hours, washing with water, drying at 100°C for 8 hours and calcining at 550°C for 6 hours, to obtain a cylindrical catalyst precursor B.
(4) 40 g of the cylindrical catalyst precursor B of step (3), ammonium fluoride and 3 mol/L piperidine solution were subjected to mixing at a mass ratio of 1:0.1:10, and reacting at 170°C for 24 hours. The mixture was subjected to washing with water and drying at 100°C for 8 hours, to obtain a Ti-MWW molecular sieve catalyst, which was recorded as S2.

The catalyst S2 was subjected to testings as described above.

An X-ray photoelectron spectrum of the catalyst S2 showed peaks at 459.1, 460.1, 465.0 and 465.7 eV.

A UV Raman spectrum of the catalyst S2 showed peaks at 341, 487, 702 and 1094 cm⁻¹. The intensity of the peak at 702 cm⁻¹ was 10 times that of the peak at 341 cm⁻¹, and the intensity of the peak at 1094 cm⁻¹ was 10 times that of the peak at 341 cm⁻¹.

An X-ray diffraction pattern and a scanning electron microscope image of the catalyst S2 were similar to Fig. 3 and Fig. 4, respectively.

The catalyst S2 had a molar ratio of silicon-titanium of 10 and a molar ratio of boron-silicon of 0.005.

The catalyst S2 had a volume of micropores of 0.05 cm³/g, a proportion of the volume of micropores to the total volume of pores of 1.7%, and a mechanical strength of 40 N/cm.

### Example 3

(1) 90 g of Ti-MWW molecular sieve powder having a molar ratio of silicon-titanium of 120, 90 g of an amorphous binder (the amorphous binder comprised 75 g of a silica sol containing silica in a mass fraction of 40% and 15 g of boric acid), 9 g of cellulose and 36 g of ammonium fluoride were mixed under mechanical stirring, to which 80 g of water was added. After stirring and kneading for 4 hours, obtained was a solid mixture, which was subjected to shaping by mechanical extruding, drying at 100 °C for 8 hours, and calcining at 550 °C for 6 hours, to obtain a cylindrical shaped product.
(2) 90 g of the cylindrical shaped product of step (1) was placed over 60 g of a 3 mol/L piperidine solution without contacting the same, to be subjected to crystallizing at 170 °C for 2 days in a closed environment. The product was subjected to washing with water and drying at 100 °C for 8 hours, to obtain a cylindrical catalyst precursor A.
(3) 60 g of the cylindrical catalyst precursor A of step (2) and 2 mol/L nitric acid solution were subjected to mixing at a mass ratio of 1:50, reacting at 80°C for 24 hours, washing with water, drying at 100°C for 8 hours and calcining at 550°C for 6 hours, to obtain a cylindrical catalyst precursor B.
(4) 40 g of the cylindrical catalyst precursor B of step (3), ammonium fluoride and 3 mol/L piperidine solution were subjected to mixing at a mass ratio of 1:0.1:10, and reacting at 170°C for 24 hours. The mixture was subjected to washing with water and drying at 100°C for 8 hours, to obtain a Ti-MWW molecular sieve catalyst, which was recorded as S3.

The catalyst S3 was subjected to testings as described above.

An X-ray photoelectron spectrum of the catalyst S3 showed peaks at 458.7, 460.4, 464.6 and 466.0 eV.

A UV Raman spectrum of the catalyst S3 showed peaks at 345, 482, 696 and 1099 cm⁻¹. The intensity of the peak at 696cm⁻¹ was 0.5 times that of the peak at 345 cm⁻¹, and the intensity of the peak at 1099 cm⁻¹ was 0.5 times that of the peak at 345 cm⁻¹.

An X-ray diffraction pattern and a scanning electron microscope image of the catalyst S3 were similar to Fig. 3 and Fig. 4, respectively.

The catalyst S3 had a molar ratio of silicon-titanium of 200 and a molar ratio of boron-silicon of 0.03.

The catalyst S3 had a volume of micropores of 0.07 cm³/g, a proportion of the volume of micropores to the total volume of pores of 2.3%, and a mechanical strength of 80 N/cm.

### Example 4

(1) 90 g of Ti-MWW molecular sieve powder having a molar ratio of silicon-titanium of 20, 9 g of an amorphous binder (the amorphous binder comprised 8 g of a silica sol containing silica in a mass fraction of 25% and 1 g of sodium tetraborate), 3 g of sesbania powder and 9 g of sodium fluoride were mixed under mechanical stirring, to which 120 g of water was added. After stirring and kneading for 4 hours, obtained was a solid mixture, which was subjected to shaping by mechanical extruding, drying at 100 °C for 8 hours, and calcining at 450 °C for 12 hours, to obtain a cylindrical shaped product.
(2) 90 g of the cylindrical shaped product of step (1) was placed over 60 g of a 3 mol/L piperidine solution without contacting the same, to be subjected to crystallizing at 170 °C for 2 days in a closed environment. The product was subjected to washing with water and drying at 100 °C for 8 hours, to obtain a cylindrical catalyst precursor A.
(3) 60 g of the cylindrical catalyst precursor A of step (2) and 2 mol/L nitric acid solution were subjected to mixing at a mass ratio of 1:50, reacting at 80°C for 24 hours, washing with water, drying at 100°C for 8 hours and calcining at 550°C for 6 hours, to obtain a cylindrical catalyst precursor B.
(4) 40 g of the cylindrical catalyst precursor B of step (3), ammonium fluoride and 3 mol/L piperidine solution were subjected to mixing at a mass ratio of 1:0.1:10, and reacting at 170°C for 24 hours. The mixture was subjected to washing with water and drying at 100°C for 8 hours, to obtain a Ti-MWW molecular sieve catalyst, which was recorded as S4.

The catalyst S4 was subjected to testings as described above.

An X-ray photoelectron spectrum of the catalyst S4 showed peaks at 458.7, 460.5, 464.6 and 466.1 eV.

A UV Raman spectrum of the catalyst S4 showed peaks at 347, 480, 695 and 1101 cm⁻¹. The intensity of the peak at 695 cm⁻¹ was 8.2 times that of the peak at 347 cm⁻¹, and the intensity of the peak at 1101 cm⁻¹ was 7.8 times that of the peak at 347 cm⁻¹.

An X-ray diffraction pattern and a scanning electron microscope image of the catalyst S4 were similar to Fig. 3 and Fig. 4, respectively.

The catalyst S4 had a molar ratio of silicon-titanium of 25 and a molar ratio of boron-silicon of 0.003.

The catalyst S4 had a volume of micropores of 0.06 cm³/g, a proportion of the volume of micropores to the total volume of pores of 2%, and a mechanical strength of 30 N/cm.

### Example 5

(1) 90 g of Ti-MWW molecular sieve powder having a molar ratio of silicon-titanium of 20, 90 g of an amorphous binder (the amorphous binder comprised 30 g of white carbon black, 30 g of boric acid, and 30 g of aluminum hydroxide), 3 g of sesbania powder and 9 g of sodium fluoride were mixed under mechanical stirring, to which 120 g of water was added. After stirring and kneading for 4 hours, obtained was a solid mixture, which was subjected to shaping by mechanical extruding, drying at 100 °C for 8 hours, and calcining at 650 °C for 4 hours, to obtain a cylindrical shaped product.
(2) 90 g of the cylindrical shaped product of step (1) was placed over 60 g of a 3 mol/L piperidine solution without contacting the same, to be subjected to crystallizing at 170 °C for 2 days in a closed environment. The product was subjected to washing with water and drying at 100 °C for 8 hours, to obtain a cylindrical catalyst precursor A.
(3) 60 g of the cylindrical catalyst precursor A of step (2) and 2 mol/L nitric acid solution were subjected to mixing at a mass ratio of 1:50, reacting at 80°C for 24 hours, washing with water, drying at 100°C for 8 hours and calcining at 550°C for 6 hours, to obtain a cylindrical catalyst precursor B.
(4) 40 g of the cylindrical catalyst precursor B of step (3), ammonium fluoride and 3 mol/L piperidine solution were subjected to mixing at a mass ratio of 1:0.1:10, and reacting at 170°C for 24 hours. The mixture was subjected to washing with water and drying at 100°C for 8 hours, to obtain a Ti-MWW molecular sieve catalyst, which was recorded as S5.

The catalyst S5 was subjected to testings as described above.

An X-ray photoelectron spectrum of the catalyst S5 showed peaks at 459.0, 460.2, 464.9 and 465.8 eV.

A UV Raman spectrum of the catalyst S5 showed peaks at 341, 486, 701 and 1095 cm⁻¹. The intensity of the peak at 701 cm⁻¹ was 2.9 times that of the peak at 341 cm⁻¹, and the intensity of the peak at 1095 cm⁻¹ was 2.6 times that of the peak at 341 cm⁻¹.

An X-ray diffraction pattern and a scanning electron microscope image of the catalyst S5 were similar to Fig. 3 and Fig. 4, respectively.

The catalyst S5 had a molar ratio of silicon-titanium of 60, a molar ratio of boron-silicon of 0.1 and a molar ratio of alunimum-silicon of 0.1.

The catalyst S5 had a volume of micropores of 0.1 cm³/g, a proportion of the volume of micropores to the total volume of pores of 4%, and a mechanical strength of 77 N/cm.

### Example 6

(1) 90 g of Ti-MWW molecular sieve powder having a molar ratio of silicon-titanium of 20, 90 g of an amorphous binder (the amorphous binder comprised 75 g of a silica sol containing silica in a mass fraction of 40% and 15 g of aluminum hydroxide), 3 g of sesbania powder and 9 g of sodium fluoride were mixed under mechanical stirring, to which 80 g of water was added. After stirring and kneading for 4 hours, obtained was a solid mixture, which was subjected to shaping by mechanical extruding, drying at 100 °C for 8 hours, and calcining at 550 °C for 6 hours, to obtain a cylindrical shaped product.
(2) 90 g of the cylindrical shaped product of step (1) was placed over 60 g of a 3 mol/L piperidine solution without contacting the same, to be subjected to crystallizing at 170 °C for 2 days in a closed environment. The product was subjected to washing with water and drying at 100 °C for 8 hours, to obtain a cylindrical catalyst precursor A.
(3) 60 g of the cylindrical catalyst precursor A of step (2) and 2 mol/L nitric acid solution were subjected to mixing at a mass ratio of 1:50, reacting at 80°C for 24 hours, washing with water, drying at 100°C for 8 hours and calcining at 550°C for 6 hours, to obtain a cylindrical catalyst precursor B.
(4) 40 g of the cylindrical catalyst precursor B of step (3), ammonium fluoride and 3 mol/L piperidine solution were subjected to mixing at a mass ratio of 1:0.1:10, and reacting at 170°C for 24 hours. The mixture was subjected to washing with water and drying at 100°C for 8 hours, to obtain a Ti-MWW molecular sieve catalyst, which was recorded as S6.

The catalyst S6 was subjected to testings as described above.

An X-ray photoelectron spectrum of the catalyst S6 showed peaks at 459.1, 460.1, 465.0 and 465.7 eV.

A UV Raman spectrum of the catalyst S6 showed peaks at 339, 488, 703 and 1093 cm⁻¹. The intensity of the peak at 703 cm⁻¹ was 4.9 times that of the peak at 339 cm⁻¹, and the intensity of the peak at 1093 cm⁻¹ was 5.2 times that of the peak at 339 cm⁻¹.

An X-ray diffraction pattern and a scanning electron microscope image of the catalyst S6 were similar to Fig. 3 and Fig. 4, respectively.

The catalyst S6 had a molar ratio of silicon-titanium of 37 and a molar ratio of aluminum-silicon of 0.05.

The catalyst S6 had a volume of micropores of 0.12 cm³/g, a proportion of the volume of micropores to the total volume of pores of 6%, and a mechanical strength of 90 N/cm.

### Example 7

(1) 90 g of Ti-MWW molecular sieve powder having a molar ratio of silicon-titanium of 20, 90 g of an amorphous binder (the amorphous binder comprised 75 g of a silica sol containing silica in a mass fraction of 40% and 15 g of boric acid), 3 g of sesbania powder and 9 g of sodium fluoride were mixed under mechanical stirring, to which 80 g of water was added. After stirring and kneading for 4 hours, obtained was a solid mixture, which was subjected to shaping by spheronizing, drying at 100 °C for 8 hours, and calcining at 550 °C for 6 hours, to obtain a spherical shaped product.
(2) 90 g of the spherical shaped product of step (1) was placed over 60 g of a 3 mol/L piperidine solution without contacting the same, to be subjected to crystallizing at 170 °C for 2 days in a closed environment. The product was subjected to washing with water and drying at 100 °C for 8 hours, to obtain a spherical catalyst precursor A.
(3) 60 g of the spherical catalyst precursor A of step (2) and 2 mol/L nitric acid solution were subjected to mixing at a mass ratio of 1:50, reacting at 80°C for 24 hours, washing with water, drying at 100°C for 8 hours and calcining at 550°C for 6 hours, to obtain a spherical catalyst precursor B.
(4) 40 g of the spherical catalyst precursor B of step (3), ammonium fluoride and 3 mol/L piperidine solution were subjected to mixing at a mass ratio of 1:0.1:10, and reacting at 170°C for 24 hours. The mixture was subjected to washing with water and drying at 100°C for 8 hours, to obtain a Ti-MWW molecular sieve catalyst, which was recorded as S7.

The catalyst S7 was subjected to testings as described above.

An X-ray photoelectron spectrum of the catalyst S7 showed peaks at 458.9, 460.3, 464.8 and 465.9 eV.

A UV Raman spectrum of the catalyst S7 showed peaks at 343, 484, 699 and 1097 cm⁻¹. The intensity of the peak at 699 cm⁻¹ was 5.3 times that of the peak at 343 cm⁻¹, and the intensity of the peak at 1097 cm⁻¹ was 5.2 times that of the peak at 343 cm⁻¹.

An X-ray diffraction pattern and a scanning electron microscope image of the catalyst S7 were similar to Fig. 3 and Fig. 4, respectively.

The catalyst S7 had a molar ratio of silicon-titanium of 34 and a molar ratio of boron-silicon of 0.014.

The catalyst S7 had a volume of micropores of 0.09 cm³/g, a proportion of the volume of micropores to the total volume of pores of 3.6%, and a mechanical strength of 80 N/cm.

### Example 8

(1) 90 g of Ti-MWW molecular sieve powder having a molar ratio of silicon-titanium of 20, 90 g of an amorphous binder (the amorphous binder comprised 75 g of a silica sol containing silica in a mass fraction of 40% and 15 g of boric acid), 3 g of sesbania powder and 9 g of sodium fluoride were mixed under mechanical stirring, to which 80 g of water was added. After stirring and kneading for 4 hours, obtained was a solid mixture, which was subjected to shaping by mechanical extruding, drying at 100 °C for 8 hours, and calcining at 550 °C for 6 hours, to obtain a cylindrical shaped product.
(2) 90 g of the cylindrical shaped product of step (1) was placed over 900 g of a 0.3 mol/L piperidine solution without contacting the same, to be subjected to crystallizing at 170 °C for 2 days in a closed environment. The product was subjected to washing with water and drying at 100 °C for 8 hours, to obtain a cylindrical catalyst precursor A.
(3) 60 g of the cylindrical catalyst precursor A of step (2) and 2 mol/L nitric acid solution were subjected to mixing at a mass ratio of 1:50, reacting at 80°C for 24 hours, washing with water, drying at 100°C for 8 hours and calcining at 550°C for 6 hours, to obtain a cylindrical catalyst precursor B.
(4) 40 g of the cylindrical catalyst precursor B of step (3), ammonium fluoride and 3 mol/L piperidine solution were subjected to mixing at a mass ratio of 1:0.1:10, and reacting at 170°C for 24 hours. The mixture was subjected to washing with water and drying at 100°C for 8 hours, to obtain a Ti-MWW molecular sieve catalyst, which was recorded as S8.

The catalyst S8 was subjected to testings as described above.

An X-ray photoelectron spectrum of the catalyst S8 showed peaks at 459.0, 460.2, 464.9 and 465.8 eV.

A UV Raman spectrum of the catalyst S8 showed peaks at 341, 486, 701 and 1095 cm⁻¹. The intensity of the peak at 701 cm⁻¹ was 4.8 times that of the peak at 341 cm⁻¹, and the intensity of the peak at 1095 cm⁻¹ was 5.9 times that of the peak at 341 cm⁻¹.

An X-ray diffraction pattern and a scanning electron microscope image of the catalyst S8 were similar to Fig. 3 and Fig. 4, respectively.

The catalyst S8 had a molar ratio of silicon-titanium of 32 and a molar ratio of boron-silicon of 0.017.

The catalyst S8 had a volume of micropores of 0.07 cm³/g, a proportion of the volume of micropores to the total volume of pores of 2.3%, and a mechanical strength of 56 N/cm.

### Example 9

(1) 90 g of Ti-MWW molecular sieve powder having a molar ratio of silicon-titanium of 20, 90 g of an amorphous binder (the amorphous binder comprised 75 g of a silica sol containing silica in a mass fraction of 40% and 15 g of boric acid), 3 g of sesbania powder and 9 g of sodium fluoride were mixed under mechanical stirring, to which 80 g of water was added. After stirring and kneading for 4 hours, obtained was a solid mixture, which was subjected to shaping by mechanical extruding, drying at 100 °C for 8 hours, and calcining at 550 °C for 6 hours, to obtain a cylindrical shaped product.
(2) 90 g of the cylindrical shaped product of step (1) was placed over 9 g of a 15 mol/L piperidine solution without contacting the same, to be subjected to crystallizing at 170 °C for 2 days in a closed environment. The product was subjected to washing with water and drying at 100 °C for 8 hours, to obtain a cylindrical catalyst precursor A.
(3) 60 g of the cylindrical catalyst precursor A of step (2) and 2 mol/L nitric acid solution were subjected to mixing at a mass ratio of 1:50, reacting at 80°C for 24 hours, washing with water, drying at 100°C for 8 hours and calcining at 550°C for 6 hours, to obtain a cylindrical catalyst precursor B.
(4) 40 g of the cylindrical catalyst precursor B of step (3), ammonium fluoride and 3 mol/L piperidine solution were subjected to mixing at a mass ratio of 1:0.1:10, and reacting at 170°C for 24 hours. The mixture was subjected to washing with water and drying at 100°C for 8 hours, to obtain a Ti-MWW molecular sieve catalyst, which was recorded as S9.

The catalyst S9 was subjected to testings as described above.

An X-ray photoelectron spectrum of the catalyst S9 showed peaks at 458.9, 460.4, 464.8 and 466.0 eV.

A UV Raman spectrum of the catalyst S9 showed peaks at 345, 483, 698 and 1099 cm⁻¹. The intensity of the peak at 698 cm⁻¹ was 5.7 times that of the peak at 345 cm⁻¹, and the intensity of the peak at 1099 cm⁻¹ was 4.9 times that of the peak at 345 cm⁻¹.

An X-ray diffraction pattern and a scanning electron microscope image of the catalyst S9 were similar to Fig. 3 and Fig. 4, respectively.

The catalyst S9 had a molar ratio of silicon-titanium of 34 and a molar ratio of boron-silicon of 0.016.

The catalyst S9 had a volume of micropores of 0.08 cm³/g, a proportion of the volume of micropores to the total volume of pores of 3.2%, and a mechanical strength of 64 N/cm.

### Example 10

(1) 90 g of Ti-MWW molecular sieve powder having a molar ratio of silicon-titanium of 20, 90 g of an amorphous binder (the amorphous binder comprised 75 g of a silica sol containing silica in a mass fraction of 40% and 15 g of boric acid), 3 g of sesbania powder and 9 g of sodium fluoride were mixed under mechanical stirring, to which 80 g of water was added. After stirring and kneading for 4 hours, obtained was a solid mixture, which was subjected to shaping by mechanical extruding, drying at 100 °C for 8 hours, and calcining at 550 °C for 6 hours, to obtain a cylindrical shaped product.
(2) 90 g of the cylindrical shaped product of step (1) was placed over 60 g of a 3 mol/L hexamethyleneimine solution without contacting the same, to be subjected to crystallizing at 130 °C for 9 days in a closed environment. The product was subjected to washing with water and drying at 100 °C for 8 hours, to obtain a cylindrical catalyst precursor A.
(3) 60 g of the cylindrical catalyst precursor A of step (2) and 2 mol/L nitric acid solution were subjected to mixing at a mass ratio of 1:50, reacting at 80°C for 24 hours, washing with water, drying at 100°C for 8 hours and calcining at 550°C for 6 hours, to obtain a cylindrical catalyst precursor B.
(4) 40 g of the cylindrical catalyst precursor B of step (3), ammonium fluoride and 3 mol/L piperidine solution were subjected to mixing at a mass ratio of 1:0.1:10, and reacting at 170°C for 24 hours. The mixture was subjected to washing with water and drying at 100°C for 8 hours, to obtain a Ti-MWW molecular sieve catalyst, which was recorded as S10.

The catalyst S10 was subjected to testings as described above.

An X-ray photoelectron spectrum of the catalyst S10 showed peaks at 459.0, 460.2, 464.9 and 465.8 eV.

A UV Raman spectrum of the catalyst S10 showed peaks at 341, 486, 701 and 1095 cm⁻¹. The intensity of the peak at 701 cm⁻¹ was 4.5 times that of the peak at 341 cm⁻¹, and the intensity of the peak at 1095 cm⁻¹ was 4.9 times that of the peak at 341 cm⁻¹.

An X-ray diffraction pattern and a scanning electron microscope image of the catalyst S10 were similar to Fig. 3 and Fig. 4, respectively.

The catalyst S10 had a molar ratio of silicon-titanium of 42 and a molar ratio of boron-silicon of 0.02.

The catalyst S10 had a volume of micropores of 0.06 cm³/g, a proportion of the volume of micropores to the total volume of pores of 2%, and a mechanical strength of 45 N/cm.

### Example 11

(1) 90 g of Ti-MWW molecular sieve powder having a molar ratio of silicon-titanium of 20, 90 g of an amorphous binder (the amorphous binder comprised 75 g of a silica sol containing silica in a mass fraction of 40% and 15 g of boric acid), 3 g of sesbania powder and 9 g of sodium fluoride were mixed under mechanical stirring, to which 80 g of water was added. After stirring and kneading for 4 hours, obtained was a solid mixture, which was subjected to shaping by mechanical extruding, drying at 100 °C for 8 hours, and calcining at 550 °C for 6 hours, to obtain a cylindrical shaped product.
(2) 90 g of the cylindrical shaped product of step (1) was placed over 60 g of a 3 mol/L hexamethyleneimine solution without contacting the same, to be subjected to crystallizing at 190 °C for 1 day in a closed environment. The product was subjected to washing with water and drying at 100 °C for 8 hours, to obtain a cylindrical catalyst precursor A.
(3) 60 g of the cylindrical catalyst precursor A of step (2) and 2 mol/L nitric acid solution were subjected to mixing at a mass ratio of 1:50, reacting at 80°C for 24 hours, washing with water, drying at 100°C for 8 hours and calcining at 550°C for 6 hours, to obtain a cylindrical catalyst precursor B.
(4) 40 g of the cylindrical catalyst precursor B of step (3), ammonium fluoride and 3 mol/L piperidine solution were subjected to mixing at a mass ratio of 1:0.1:10, and reacting at 170°C for 24 hours. The mixture was subjected to washing with water and drying at 100°C for 8 hours, to obtain a Ti-MWW molecular sieve catalyst, which was recorded as S11.

The catalyst S11 was subjected to testings as described above.

An X-ray photoelectron spectrum of the catalyst S11 showed peaks at 458.9, 460.4, 464.8 and 466.0 eV.

A UV Raman spectrum of the catalyst S11 showed peaks at 345, 482, 698 and 1099 cm⁻¹. The intensity of the peak at 698 cm⁻¹ was 6 times that of the peak at 345 cm⁻¹, and the intensity of the peak at 1099 cm⁻¹ was 5 times that of the peak at 345 cm⁻¹.

An X-ray diffraction pattern and a scanning electron microscope image of the catalyst S11 were similar to Fig. 3 and Fig. 4, respectively.

The catalyst S11 had a molar ratio of silicon-titanium of 30 and a molar ratio of boron-silicon of 0.01.

The catalyst S11 had a volume of micropores of 0.07 cm³/g, a proportion of the volume of micropores to the total volume of pores of 2.3%, and a mechanical strength of 69 N/cm.

### Example 12

(1) 90 g of Ti-MWW molecular sieve powder having a molar ratio of silicon-titanium of 20, 90 g of an amorphous binder (the amorphous binder comprised 75 g of a silica sol containing silica in a mass fraction of 40% and 15 g of boric acid), 3 g of sesbania powder and 9 g of sodium fluoride were mixed under mechanical stirring, to which 80 g of water was added. After stirring and kneading for 4 hours, obtained was a solid mixture, which was subjected to shaping by mechanical extruding, drying at 100 °C for 8 hours, and calcining at 550 °C for 6 hours, to obtain a cylindrical shaped product.
(2) 90 g of the cylindrical shaped product of step (1) was placed over 60 g of a 3 mol/L piperidine solution without contacting the same, to be subjected to crystallizing at 170 °C for 2 days in a closed environment. The product was subjected to washing with water and drying at 100 °C for 8 hours, to obtain a cylindrical catalyst precursor A.
(3) 60 g of the cylindrical catalyst precursor A of step (2) and 12 mol/L hydrochloric acid solution were subjected to mixing at a mass ratio of 1:10, reacting at 130°C for 4 hours, washing with water, drying at 100°C for 8 hours and calcining at 450°C for 12 hours, to obtain a cylindrical catalyst precursor B.
(4) 40 g of the cylindrical catalyst precursor B of step (3), ammonium fluoride and 3 mol/L piperidine solution were subjected to mixing at a mass ratio of 1:0.1:10, and reacting at 170°C for 24 hours. The mixture was subjected to washing with water and drying at 100°C for 8 hours, to obtain a Ti-MWW molecular sieve catalyst, which was recorded as S12.

The catalyst S12 was subjected to testings as described above.

An X-ray photoelectron spectrum of the catalyst S12 showed peaks at 458.9, 460.5, 464.8 and 466.1 eV.

A UV Raman spectrum of the catalyst S12 showed peaks at 344, 481, 698 and 1100 cm⁻¹. The intensity of the peak at 698 cm⁻¹ was 3.3 times that of the peak at 344 cm⁻¹, and the intensity of the peak at 1100 cm⁻¹ was 2.4 times that of the peak at 344 cm⁻¹.

An X-ray diffraction pattern and a scanning electron microscope image of the catalyst S12 were similar to Fig. 3 and Fig. 4, respectively.

The catalyst S12 had a molar ratio of silicon-titanium of 57 and a molar ratio of boron-silicon of 0.004.

The catalyst S12 had a volume of micropores of 0.1 cm³/g, a proportion of the volume of micropores to the total volume of pores of 4%, and a mechanical strength of 48 N/cm.

### Example 13

(1) 90 g of Ti-MWW molecular sieve powder having a molar ratio of silicon-titanium of 20, 90 g of an amorphous binder (the amorphous binder comprised 75 g of a silica sol containing silica in a mass fraction of 40% and 15 g of boric acid), 3 g of sesbania powder and 9 g of sodium fluoride were mixed under mechanical stirring, to which 80 g of water was added. After stirring and kneading for 4 hours, obtained was a solid mixture, which was subjected to shaping by mechanical extruding, drying at 100 °C for 8 hours, and calcining at 550 °C for 6 hours, to obtain a cylindrical shaped product.
(2) 90 g of the cylindrical shaped product of step (1) was placed over 60 g of a 3 mol/L piperidine solution without contacting the same, to be subjected to crystallizing at 170 °C for 2 days in a closed environment. The product was subjected to washing with water and drying at 100 °C for 8 hours, to obtain a cylindrical catalyst precursor A.
(3) 60 g of the cylindrical catalyst precursor A of step (2) and 0.3 mol/L oxalic acid solution were subjected to mixing at a mass ratio of 1:80, reacting at 60°C for 48 hours, washing with water, drying at 100°C for 8 hours and calcining at 650°C for 4 hours, to obtain a cylindrical catalyst precursor B.
(4) 40 g of the cylindrical catalyst precursor B of step (3), ammonium fluoride and 3 mol/L piperidine solution were subjected to mixing at a mass ratio of 1:0.1:10, and reacting at 170°C for 24 hours. The mixture was subjected to washing with water and drying at 100°C for 8 hours, to obtain a Ti-MWW molecular sieve catalyst, which was recorded as S13.

The catalyst S13 was subjected to testings as described above.

An X-ray photoelectron spectrum of the catalyst S13 showed peaks at 459.1, 460.1, 465.0 and 465.7 eV.

A UV Raman spectrum of the catalyst S13 showed peaks at 341, 488, 702 and 1093 cm⁻¹. The intensity of the peak at 702 cm⁻¹ was 5.9 times that of the peak at 341 cm⁻¹, and the intensity of the peak at 1093 cm⁻¹ was 5.7 times that of the peak at 341 cm⁻¹.

An X-ray diffraction pattern and a scanning electron microscope image of the catalyst S13 were similar to Fig. 3 and Fig. 4, respectively.

The catalyst S13 had a molar ratio of silicon-titanium of 27 and a molar ratio of boron-silicon of 0.08.

The catalyst S13 had a volume of micropores of 0.05 cm³/g, a proportion of the volume of micropores to the total volume of pores of 1.7%, and a mechanical strength of 60 N/cm.

### Example 14

(1) 90 g of Ti-MWW molecular sieve powder having a molar ratio of silicon-titanium of 20, 90 g of an amorphous binder (the amorphous binder comprised 75 g of a silica sol containing silica in a mass fraction of 40% and 15 g of boric acid), 3 g of sesbania powder and 9 g of sodium fluoride were mixed under mechanical stirring, to which 80 g of water was added. After stirring and kneading for 4 hours, obtained was a solid mixture, which was subjected to shaping by mechanical extruding, drying at 100 °C for 8 hours, and calcining at 550 °C for 6 hours, to obtain a cylindrical shaped product.
(2) 90 g of the cylindrical shaped product of step (1) was placed over 60 g of a 3 mol/L piperidine solution without contacting the same, to be subjected to crystallizing at 170 °C for 2 days in a closed environment. The product was subjected to washing with water and drying at 100 °C for 8 hours, to obtain a cylindrical catalyst precursor A.
(3) 60 g of the cylindrical catalyst precursor A of step (2) and 2 mol/L nitric acid solution were subjected to mixing at a mass ratio of 1:50, reacting at 80°C for 24 hours, washing with water, drying at 100°C for 8 hours and calcining at 550°C for 6 hours, to obtain a cylindrical catalyst precursor B.
(4) 40 g of the cylindrical catalyst precursor B of step (3), ammonium fluoride and 3 mol/L hexamethyleneimine solution were subjected to mixing at a mass ratio of 1:0.2:20, and reacting at 170°C for 48 hours. The mixture was subjected to washing with water and drying at 100°C for 8 hours, to obtain a Ti-MWW molecular sieve catalyst, which was recorded as S14.

The catalyst S14 was subjected to testings as described above.

An X-ray photoelectron spectrum of the catalyst S14 showed peaks at 458.9, 460.3, 464.8 and 465.9 eV.

A UV Raman spectrum of the catalyst S14 showed peaks at 342, 484, 700 and 1097 cm⁻¹. The intensity of the peak at 700 cm⁻¹ was 5 times that of the peak at 342 cm⁻¹, and the intensity of the peak at 1097 cm⁻¹ was 4.8 times that of the peak at 342 cm⁻¹.

An X-ray diffraction pattern and a scanning electron microscope image of the catalyst S14 were similar to Fig. 3 and Fig. 4, respectively.

The catalyst S14 had a molar ratio of silicon-titanium of 39 and a molar ratio of boron-silicon of 0.018.

The catalyst S14 had a volume of micropores of 0.08 cm³/g, a proportion of the volume of micropores to the total volume of pores of 3.2%, and a mechanical strength of 62 N/cm.

### Example 15

(1) 90 g of Ti-MWW molecular sieve powder having a molar ratio of silicon-titanium of 20, 90 g of an amorphous binder (the amorphous binder comprised 75 g of a silica sol containing silica in a mass fraction of 40% and 15 g of boric acid), 3 g of sesbania powder and 9 g of sodium fluoride were mixed under mechanical stirring, to which 80 g of water was added. After stirring and kneading for 4 hours, obtained was a solid mixture, which was subjected to shaping by mechanical extruding, drying at 100 °C for 8 hours, and calcining at 550 °C for 6 hours, to obtain a cylindrical shaped product.
(2) 90 g of the cylindrical shaped product of step (1) was placed over 60 g of a 3 mol/L piperidine solution without contacting the same, to be subjected to crystallizing at 170 °C for 2 days in a closed environment. The product was subjected to washing with water and drying at 100 °C for 8 hours, to obtain a cylindrical catalyst precursor A.
(3) 60 g of the cylindrical catalyst precursor A of step (2) and 2 mol/L nitric acid solution were subjected to mixing at a mass ratio of 1:50, reacting at 80°C for 24 hours, washing with water, drying at 100°C for 8 hours and calcining at 550°C for 6 hours, to obtain a cylindrical catalyst precursor B.
(4) 40 g of the cylindrical catalyst precursor B of step (3), potassium fluoride and 15 mol/L piperidine solution were subjected to mixing at a mass ratio of 1:0.4:2, and reacting at 190°C for 4 hours. The mixture was subjected to washing with water and drying at 100°C for 8 hours, to obtain a Ti-MWW molecular sieve catalyst, which was recorded as S15.

The catalyst S15 was subjected to testings as described above.

An X-ray photoelectron spectrum of the catalyst S15 showed peaks at 459.0, 460.2, 464.9 and 465.8 eV.

A UV Raman spectrum of the catalyst S15 showed peaks at 342, 485, 701 and 1095 cm⁻¹. The intensity of the peak at 701 cm⁻¹ was 6.8 times that of the peak at 342 cm⁻¹, and the intensity of the peak at 1095 cm⁻¹ was 3.2 times that of the peak at 342 cm⁻¹.

An X-ray diffraction pattern and a scanning electron microscope image of the catalyst S15 were similar to Fig. 3 and Fig. 4, respectively.

The catalyst S15 had a molar ratio of silicon-titanium of 37 and a molar ratio of boron-silicon of 0.025.

The catalyst S15 had a volume of micropores of 0.15 cm³/g, a proportion of the volume of micropores to the total volume of pores of 7.5%, and a mechanical strength of 64 N/cm.

### Example 16

(1) 90 g of Ti-MWW molecular sieve powder having a molar ratio of silicon-titanium of 20, 90 g of an amorphous binder (the amorphous binder comprised 75 g of a silica sol containing silica in a mass fraction of 40% and 15 g of boric acid), 3 g of sesbania powder and 9 g of sodium fluoride were mixed under mechanical stirring, to which 80 g of water was added. After stirring and kneading for 4 hours, obtained was a solid mixture, which was subjected to shaping by mechanical extruding, drying at 100 °C for 8 hours, and calcining at 550 °C for 6 hours, to obtain a cylindrical shaped product.
(2) 90 g of the cylindrical shaped product of step (1) was placed over 60 g of a 3 mol/L piperidine solution without contacting the same, to be subjected to crystallizing at 170 °C for 2 days in a closed environment. The product was subjected to washing with water and drying at 100 °C for 8 hours, to obtain a cylindrical catalyst precursor A.
(3) 60 g of the cylindrical catalyst precursor A of step (2) and 2 mol/L nitric acid solution were subjected to mixing at a mass ratio of 1:50, reacting at 80°C for 24 hours, washing with water, drying at 100°C for 8 hours and calcining at 550°C for 6 hours, to obtain a cylindrical catalyst precursor B.
(4) 40 g of the cylindrical catalyst precursor B of step (3), sodium fluoride and 0.3 mol/L piperidine solution were subjected to mixing at a mass ratio of 1:0.05:20, and reacting at 130°C for 48 hours. The mixture was subjected to washing with water and drying at 100°C for 8 hours, to obtain a Ti-MWW molecular sieve catalyst, which was recorded as S16.

The catalyst S16 was subjected to testings as described above.

An X-ray photoelectron spectrum of the catalyst S16 showed peaks at 458.9, 460.3, 464.8 and 465.9 eV.

A UV Raman spectrum of the catalyst S16 showed peaks at 344, 482, 698 and 1098 cm⁻¹. The intensity of the peak at 698 cm⁻¹ was 3.3 times that of the peak at 344 cm⁻¹, and the intensity of the peak at 1098 cm⁻¹ was 6.9 times that of the peak at 344 cm⁻¹.

An X-ray diffraction pattern and a scanning electron microscope image of the catalyst S16 were similar to Fig. 3 and Fig. 4, respectively.

The catalyst S16 had a molar ratio of silicon-titanium of 36 and a molar ratio of boron-silicon of 0.01.

The catalyst S16 had a volume of micropores of 0.03 cm³/g, a proportion of the volume of micropores to the total volume of pores of 1%, and a mechanical strength of 57 N/cm.

### Working Examples 17-24

A liquid phase continuous epoxidation of propylene was operated in the presence of the Ti-MWW molecular sieve catalysts prepared in Examples 1, 2, 3, 6, 11, 12, 15 and 16 to evaluate the catalytic performance thereof.

2 g of the above Ti-MWW molecular sieve catalysts were crushed into particles having a size of 20-40 mesh and loaded into a stainless steel reaction tube, wherein both ends of the reaction tube were filled with glass beads. The reaction was operated under the conditions of: at a temperature of 40 °C and a pressure of 2.0 MPa, by feeding from a lower part and discharging from an upper part of the reaction tube. Nitrogen was used to balance the pressure of propylene to 2.5 MPa to ensure that propylene was completely liquefied. The feed solution of propylene was fed separately and recorded as feed solution A. An aqueous solution with a mass fraction of hydrogen peroxide of 30% was used to prepare an aqueous solution of piperidine in hydrogen peroxide with a concentration of 15 ppm, which was mixed with a solvent acetonitrile. The mixture was recorded as feed solution B. The two feed solutions were fed separately by plunger pumps, and they were premixed before entering the reaction tube. A sample of the mixed feed solutions entering the reaction tube was collected and tested as described above. **In** the stream of the mixed feed solutions, propylene was in a mass fraction of 18.7%, acetonitrile was in a mass fraction of 61.0%, wherein the molar ratio of propylene to hydrogen peroxide was 1:0.4. The flow rate of the catalyst per unit mass of the total feed solutions was 6 mL·g_{cat}.⁻¹·h⁻¹. A sample of reaction liquid out of the reaction tube was collected and tested as described above. The results were shown in Table 1.

**Table 1 catalytic performance of the catalysts of Examples in a liquid phase continuous epoxidation of propylene**

| | catalyst | The conversion of hydrogen peroxide^{a}, % | The propotion of residual hydrogen peroxide^{a}, % | The selectivity for propylene oxide, % | The ratio of the main products to the by-products | The period for keeping stability of the catalyst, hour |
|---|---|---|---|---|---|---|
| W.E. 17 | S1 | 99.9 | 0.1 | 99.8 | 498.3 | 1820 |
| W.E. 18 | S2 | 100 | 0 | 99.9 | 999.2 | 2535 |
| W.E. 19 | S3 | 99.2 | 0.8 | 99.6 | 248.7 | 1248 |
| W.E. 20 | S6 | 99.5 | 0.5 | 99.7 | 332.5 | 1495 |
| W.E. 21 | S11 | 99.8 | 0.2 | 99.8 | 499.2 | 1762 |
| W.E. 22 | S12 | 99.5 | 0.5 | 99.8 | 498.6 | 1536 |
| W.E. 23 | S15 | 99.7 | 0.3 | 99.8 | 499.5 | 1653 |
| W.E. 24 | S16 | 99.8 | 0.2 | 99.8 | 499.8 | 1738 |

| | | | | | | |
|---|---|---|---|---|---|---|
| Note: a. A sample of reaction liquid was obtained when the reaction liquid was just emerged from the outlet of the reaction tube to calculate the conversion of hydrogen peroxide and the propotion of residual hydrogen peroxide | | | | | | |

### Working Examples 25-28

A liquid phase continuous epoxidation of allyl chloride was operated in the presence of the Ti-MWW molecular sieve catalysts prepared in Examples 1, 6, 11 and 16 to evaluate the catalytic performance thereof.
2 g of the Ti-MWW molecular sieve catalysts were crushed into particles having a size of 20-40 mesh and loaded into a stainless steel reaction tube, wherein both ends of the reaction tube were filled with glass beads. The reaction was operated under the conditions of: at a temperature of 60 °C and a pressure of 0.6 MPa, by feeding from a lower part and discharging from an upper part of the reaction tube. The feed solution of allyl chloride was fed separately and recorded as feed solution A. An aqueous solution with a mass fraction of hydrogen peroxide of 30% was used to prepare an aqueous solution of hexamethyleneimine in hydrogen peroxide with a concentration of 50 ppm, which was mixed with a solvent acetonitrile. The mixture was recorded as feed solution B. The two feed solutions were fed separately by plunger pumps, and they were premixed before entering the reaction tube. A sample of the mixed feed solutions entering the reaction tube was collected and tested as described above. In the stream of the mixed feed solutions, allyl chloride was in a mass fraction of 27.7%, acetonitrile was in a mass fraction of 54.2%, wherein the molar ratio of allyl chloride to hydrogen peroxide was 1:0.4. The flow rate of the catalyst per unit mass of the total feed solutions was 4 mL·g_{cat}⁻¹·h⁻¹. A sample of reaction liquid out of the reaction tube was collected and tested as described above. The results were shown in Table 2.

**Table 2 catalytic performance of the catalysts of Examples in a liquid phase continuous epoxidation of allyl chloride**

| | catalyst | The conversion of hydrogen peroxide^{a}, % | The propotion of residual hydrogen peroxide^{a}, % | The selectivity for propylene oxide, % | The ratio of the main products to the by-products | The period for keeping stability of the catalyst, hour |
|---|---|---|---|---|---|---|
| W.E. 25 | S1 | 99.8 | 0.2 | 99.7 | 332.5 | 1160 |
| W.E. 26 | S6 | 99.3 | 0.7 | 99.4 | 165.8 | 758 |
| W.E. 27 | S11 | 99.7 | 0.2 | 99.6 | 248.5 | 1076 |
| W.E. 28 | S16 | 99.7 | 0.3 | 99.5 | 199.3 | 1025 |

| | | | | | | |
|---|---|---|---|---|---|---|
| Note: a. A sample of reaction liquid was obtained when the reaction liquid was just emerged from the outlet of the reaction tube to calculate the conversion of hydrogen peroxide and the propotion of residual hydrogen peroxide. | | | | | | |

### Comparative Example 1

(1) 90 g of Ti-MWW molecular sieve powder having a molar ratio of silicon-titanium of 20, 90 g of an amorphous binder (the amorphous binder comprised 75 g of a silica sol containing silica in a mass fraction of 40% and 15 g of boric acid), 3 g of sesbania powder and 9 g of sodium fluoride were mixed under mechanical stirring, to which 80 g of water was added. After stirring and kneading for 4 hours, obtained was a solid mixture, which was subjected to shaping by mechanical extruding, drying at 100 °C for 8 hours, and calcining at 550 °C for 6 hours, to obtain a cylindrical shaped product.
(2) 60 g of the cylindrical shaped product of step (1) and 2 mol/L nitric acid solution were subjected to mixing at a mass ratio of 1:50, reacting at 80°C for 24 hours, washing with water, drying at 100°C for 8 hours and calcining at 550°C for 6 hours, to obtain a cylindrical catalyst precursor.
(3) 40 g of the cylindrical catalyst precursor of step (2), ammonium fluoride and 3 mol/L piperidine solution were subjected to mixing at a mass ratio of 1:0.1:10, and reacting at 170°C for 24 hours. The mixture was subjected to washing with water and drying at 100°C for 8 hours, to obtain a Ti-MWW molecular sieve catalyst, which was recorded as D1.

The catalyst D1 was subjected to testings as described above.

An X-ray photoelectron spectrum of D1 was shown in Fig. 5, which showed peaks at 459.5 and 465.1 eV. They were attributed to tetracoordinated titanium species.

A UV Raman spectrum of D1 was shown in Fig. 6, which only showed peaks at 491 and 1080 cm⁻¹. There was no peak attributed to the MWW structure. The peaks at 491 and 1080 cm⁻¹ were attributed to tetracoordinated titanium species. The results were mainly attributed to the fact that the catalyst still contained amorphous binders which had not been converted into components of the molecular sieve. The presence of amorphous binders shielded the molecular sieve, making it impossible to detect the MWW structure of the molecular sieve by UV Raman testing.

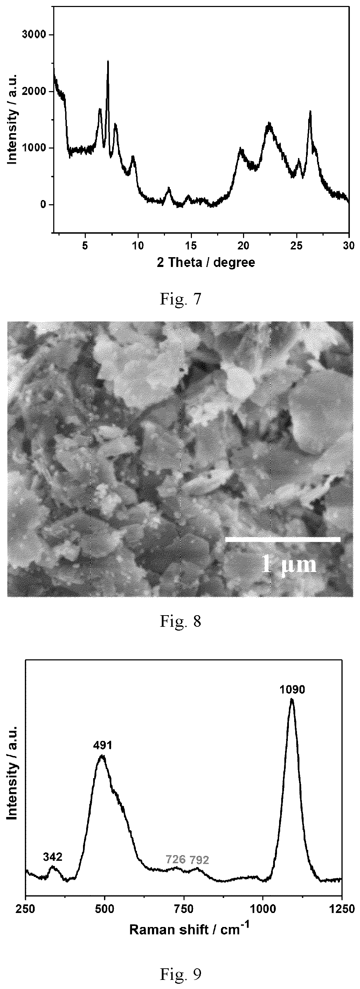

An X-ray diffraction pattern of D1 was shown in Fig. 7. There were diffraction peaks at 2θ of 3.3°, 6.6°, 7.2°, 7.9°, 9.7° and 26.1°. The intensity of the diffraction peak at 2θ of 7.2° reached about 2500, which is substantially weaker than that of S1, indicating that the bulk of D1 still was in an MWW structure but had a low crystallinity. In addition, there were stronger broad diffraction peaks in the region of 17.5-30°, further indicating the presence of amorphous species in D1.

A scanning electron microscope image was shown in Fig. 8. The catalyst showed a lamellar morphology, with nanoparticles being observed. It further indicated the presence of amorphous species in D1. It was consistent with the results shown in Fig. 7.

D1 had a molar ratio of silicon-titanium of 30 and a molar ratio of boron-silicon of 0.06.

D1 had a volume of micropores of 0.04 cm³/g, a proportion of the volume of micropores to the total volume of pores of 1.4%, and a mechanical strength of 29 N/cm.

### Comparative Example 2

(1) 90 g of Ti-MWW molecular sieve powder having a molar ratio of silicon-titanium of 20, 90 g of an amorphous binder (the amorphous binder comprised 75 g of a silica sol containing silica in a mass fraction of 40% and 15 g of boric acid), 3 g of sesbania powder and 9 g of sodium fluoride were mixed under mechanical stirring, to which 80 g of water was added. After stirring and kneading for 4 hours, obtained was a solid mixture, which was subjected to shaping by mechanical extruding, drying at 100 °C for 8 hours, and calcining at 550 °C for 6 hours, to obtain a cylindrical shaped product.
(2) 90 g of the cylindrical shaped product of step (1) was placed over 60 g of a 3 mol/L piperidine solution without contacting the same, to be subjected to crystallizing at 170 °C for 2 days in a closed environment. The product was subjected to washing with water and drying at 100 °C for 8 hours, to obtain a cylindrical catalyst precursor.
(3) 40 g of the cylindrical catalyst precursor of step (2), ammonium fluoride and 3 mol/L piperidine solution were subjected to mixing at a mass ratio of 1:0.1:10, and reacting at 170°C for 24 hours. The mixture was subjected to washing with water and drying at 100°C for 8 hours, to obtain a Ti-MWW molecular sieve catalyst, which was recorded as D2.

The catalyst D2 was subjected to testings as described above.

It was observed that an X-ray photoelectron spectrum of D2 showed peaks at 460.0 and 465.6 eV. They were attributed to framework tetracoordinated titanium species.

A UV Raman spectrum of D2 was shown in Fig. 9, which showed peaks at 342, 491 and 1090 cm⁻¹. The intensity of the peak at 1090 cm⁻¹ was 11.2 times that of the peak at 342 cm⁻¹. It indicated the presence of a large amount of framework tetracoordinated titanium species.

An X-ray diffraction pattern and a scanning electron microscope image of D2 were similar to Fig. 3 and Fig. 4, respectively.

D2 had a molar ratio of silicon-titanium of 31 and a molar ratio of boron-silicon of 0.05.

D2 had a volume of micropores of 0.08 cm³/g, a proportion of the volume of micropores to the total volume of pores of 3.2%, and a mechanical strength of 68 N/cm.

### Comparative Example 3

(1) 90 g of Ti-MWW molecular sieve powder having a molar ratio of silicon-titanium of 20, 90 g of an amorphous binder (the amorphous binder comprised 75 g of a silica sol containing silica in a mass fraction of 40% and 15 g of boric acid), 3 g of sesbania powder and 9 g of sodium fluoride were mixed under mechanical stirring, to which 80 g of water was added. After stirring and kneading for 4 hours, obtained was a solid mixture, which was subjected to shaping by mechanical extruding, drying at 100 °C for 8 hours, and calcining at 550 °C for 6 hours, to obtain a cylindrical shaped product.
(2) 90 g of the cylindrical shaped product of step (1) was placed over 60 g of a 3 mol/L piperidine solution without contacting the same, to be subjected to crystallizing at 170 °C for 2 days in a closed environment. The product was subjected to washing with water and drying at 100 °C for 8 hours, to obtain a cylindrical catalyst precursor.
(3) 60 g of the cylindrical catalyst precursor of step (2) and 2 mol/L nitric acid solution were subjected to mixing at a mass ratio of 1:50, reacting at 80°C for 24 hours, washing with water, drying at 100°C for 8 hours and calcining at 550°C for 6 hours, to obtain a Ti-MWW molecular sieve catalyst, which was recorded as D3.

The catalyst D3 was subjected to testings as described above.

It was observed that an X-ray photoelectron spectrum of D3 showed peaks at 460.0 and 465.6 eV. They were attributed to framework tetracoordinated titanium species.

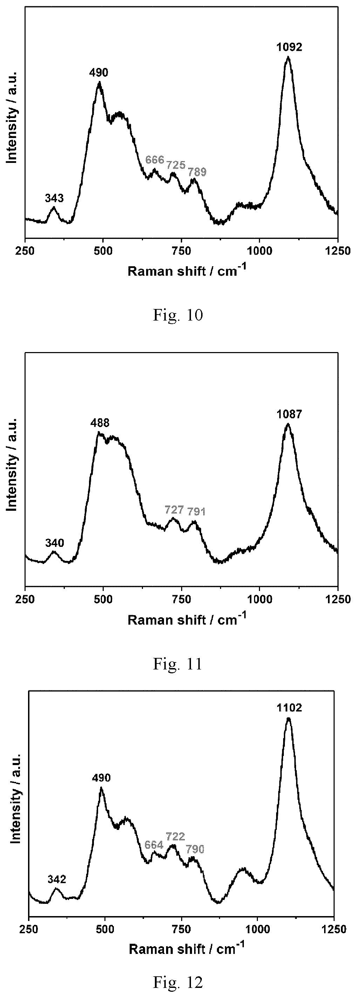

A UV Raman spectrum of D3 was shown in Fig. 10, which showed peaks at 343, 490 and 1092 cm⁻¹. The intensity of the peak at 1092 cm⁻¹ was 10.2 times that of the peak at 343 cm⁻¹. It indicated the presence of a large amount of framework tetracoordinated titanium species.

An X-ray diffraction pattern and a scanning electron microscope image of D3 were similar to Fig. 3 and Fig. 4, respectively.

D3 had a molar ratio of silicon-titanium of 36 and a molar ratio of boron-silicon of 0.017.

D3 had a volume of micropores of 0.16 cm³/g, a proportion of the volume of micropores to the total volume of pores of 7.8%, and a mechanical strength of 58 N/cm.

### Comparative Example 4

(1) 90 g of Ti-MWW molecular sieve powder having a molar ratio of silicon-titanium of 20, 90 g of an amorphous binder (the amorphous binder comprised 75 g of a silica sol containing silica in a mass fraction of 40% and 15 g of boric acid) and 3 g of sesbania powder were mixed under mechanical stirring, to which 80 g of water was added. After stirring and kneading for 4 hours, obtained was a solid mixture, which was subjected to shaping by mechanical extruding, drying at 100 °C for 8 hours, and calcining at 550 °C for 6 hours, to obtain a cylindrical shaped product.
(2) 90 g of the cylindrical shaped product of step (1) was placed over 60 g of a 3 mol/L piperidine solution without contacting the same, to be subjected to crystallizing at 170 °C for 2 days in a closed environment. The product was subjected to washing with water and drying at 100 °C for 8 hours, to obtain a cylindrical catalyst precursor A.
(3) 60 g of the cylindrical catalyst precursor A of step (2) and 2 mol/L nitric acid solution were subjected to mixing at a mass ratio of 1:50, reacting at 80°C for 24 hours, washing with water, drying at 100°C for 8 hours and calcining at 550°C for 6 hours, to obtain a cylindrical catalyst precursor B.
(4) 40 g of the cylindrical catalyst precursor B of step (3), ammonium fluoride and 3 mol/L piperidine solution were subjected to mixing at a mass ratio of 1:0.1:10, and reacting at 170°C for 24 hours. The mixture was subjected to washing with water and drying at 100°C for 8 hours, to obtain a Ti-MWW molecular sieve catalyst, which was recorded as D4.

The catalyst D4 was subjected to testings as described above.

It was observed that an X-ray photoelectron spectrum of D4 showed peaks at 459.7 and 465.4 eV. They were attributed to tetracoordinated titanium species.

A UV Raman spectrum of D4 was shown in Fig. 11, which showed peaks at 340, 488 and 1087 cm⁻¹. The intensity of the peak at 1087 cm⁻¹ was 10.9 times that of the peak at 340 cm⁻¹. It indicated the presence of a large amount of tetracoordinated titanium species.

An X-ray diffraction pattern and a scanning electron microscope image of D4 were similar to Fig. 7 and Fig. 8, respectively.

D4 had a molar ratio of silicon-titanium of 33 and a molar ratio of boron-silicon of 0.012.

D4 had a volume of micropores of 0.05 cm³/g, a proportion of the volume of micropores to the total volume of pores of 1.7%, and a mechanical strength of 36 N/cm.

### Comparative Example 5

(1) 90 g of Ti-MWW molecular sieve powder having a molar ratio of silicon-titanium of 20, 90 g of an amorphous binder (the amorphous binder comprised 75 g of a silica sol containing silica in a mass fraction of 40% and 15 g of boric acid), 3 g of sesbania powder and 9 g of sodium fluoride were mixed under mechanical stirring, to which 80 g of water was added. After stirring and kneading for 4 hours, obtained was a solid mixture, which was subjected to shaping by mechanical extruding, drying at 100 °C for 8 hours, and calcining at 550 °C for 6 hours, to obtain a cylindrical shaped product.
(2) 90 g of the cylindrical shaped product of step (1) was placed over 60 g of a 3 mol/L piperidine solution without contacting the same, to be subjected to crystallizing at 170 °C for 2 days in a closed environment. The product was subjected to washing with water and drying at 100 °C for 8 hours, to obtain a cylindrical catalyst precursor A.
(3) 60 g of the cylindrical catalyst precursor A of step (2) and 2 mol/L nitric acid solution were subjected to mixing at a mass ratio of 1:50, reacting at 80°C for 24 hours, washing with water, drying at 100°C for 8 hours and calcining at 550°C for 6 hours, to obtain a cylindrical catalyst precursor B.
(4) 40 g of the cylindrical catalyst precursor B of step (3) and 3 mol/L piperidine solution were subjected to mixing at a mass ratio of 1:10, and reacting at 170°C for 24 hours. The mixture was subjected to washing with water and drying at 100°C for 8 hours, to obtain a Ti-MWW molecular sieve catalyst, which was recorded as D5.

The catalyst D5 was subjected to testings as described above.

It was observed that an X-ray photoelectron spectrum of D5 showed peaks at 460.6 and 466.2 eV. They were attributed to tetracoordinated titanium species.

A UV Raman spectrum of D5 was shown in Fig. 12, which showed peaks at 342, 490 and 1102 cm⁻¹. The intensity of the peak at 1102 cm⁻¹ was 10.3 times that of the peak at 342 cm⁻¹. It indicated the presence of a large amount of framework tetracoordinated titanium species.

An X-ray diffraction pattern and a scanning electron microscope image of D5 were similar to Fig. 3 and Fig. 4, respectively.

D5 had a molar ratio of silicon-titanium of 36 and a molar ratio of boron-silicon of 0.017.

D5 had a volume of micropores of 0.04 cm³/g, a proportion of the volume of micropores to the total volume of pores of 1.3%, and a mechanical strength of 74 N/cm.

### Comparative Example 6

(1) 90 g of Ti-MWW molecular sieve powder having a molar ratio of silicon-titanium of 20, 90 g of an amorphous binder (the amorphous binder comprised 75 g of a silica sol containing silica in a mass fraction of 40% and 15 g of boric acid), 3 g of sesbania powder and 9 g of sodium fluoride were mixed under mechanical stirring, to which 80 g of water was added. After stirring and kneading for 4 hours, obtained was a solid mixture, which was subjected to shaping by mechanical extruding, drying at 100 °C for 8 hours, and calcining at 550 °C for 6 hours, to obtain a cylindrical shaped product, which was recorded as D6.

The catalyst D6 was subjected to testings as described above.

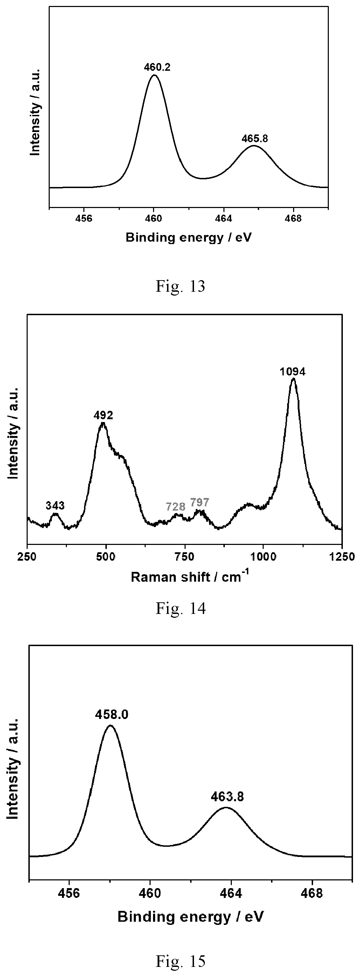

An X-ray photoelectron spectrum of D6 was shown in Fig. 13, which showed peaks at 460.2 and 465.8 eV. They were attributed to tetracoordinated titanium species.

A UV Raman spectrum of D6 was shown in Fig. 14, which showed peaks at 343, 492 and 1094 cm⁻¹. The intensity of the peak at 1094 cm⁻¹ was 9.7 times that of the peak at 343 cm⁻¹. It indicated the presence of a large amount of framework tetracoordinated titanium species.

An X-ray photoelectron spectrum and a UV Raman spectrum of a Ti-MWW molecular sieve powder were similar to Fig. 13 and Fig. 14, respectively, indicating that the shaping by mechanical extruding did not affect the state of titanium species of the catalyst D6.

An X-ray diffraction pattern and a scanning electron microscope image of D6 were similar to Fig. 7 and Fig. 8, respectively.

D6 had a molar ratio of silicon-titanium of 38 and a molar ratio of boron-silicon of 0.11.

D6 had a volume of micropores of 0.13 cm³/g, a proportion of the volume of micropores to the total volume of pores of 5.4%, and a mechanical strength of 28 N/cm.

### Comparative Example 7

(1) 90 g of Ti-MWW molecular sieve powder having a molar ratio of silicon-titanium of 20, 90 g of an amorphous binder (the amorphous binder comprised 75 g of a silica sol containing silica in a mass fraction of 40% and 15 g of boric acid), 3 g of sesbania powder and 9 g of sodium fluoride were mixed under mechanical stirring, to which 80 g of water was added. After stirring and kneading for 4 hours, obtained was a solid mixture, which was subjected to shaping by mechanical extruding, drying at 100 °C for 8 hours, and calcining at 550 °C for 6 hours, to obtain a cylindrical shaped product, which was recorded as D7.

The catalyst D7 was subjected to testings as described above.

An X-ray photoelectron spectrum of D7 was shown in Fig. 15, which showed peaks at 458.0 and 463.8 eV. They were attributed to extra-framework hexacoordinated titanium species.

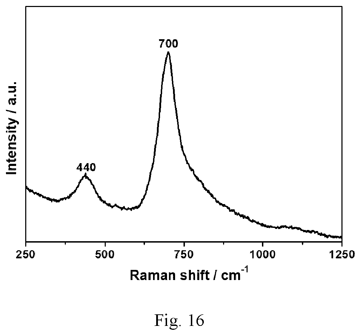

A UV Raman spectrum of D7 was shown in Fig. 16, which showed peaks at 440 and 700 cm⁻¹. They were attributed to extra-framework hexacoordinated titanium species.

An X-ray photoelectron spectrum and a UV Raman spectrum of an as-synthesized Ti-MWW molecular sieve powder were similar to Fig. 15 and Fig. 16, respectively, indicating that the shaping by mechanical extruding did not affect the state of titanium species of the catalyst D7.

An X-ray diffraction pattern and a scanning electron microscope image of D7 were similar to Fig. 7 and Fig. 8, respectively.

D7 had a molar ratio of silicon-titanium of 37 and a molar ratio of boron-silicon of 0.12.

D7 had a volume of micropores of 0.12 cm³/g, a proportion of the volume of micropores to the total volume of pores of 5.3%, and a mechanical strength of 26 N/cm.

### Comparative Working Examples 8-11

Working Examples 17-24 were repeated with the Ti-MWW molecular sieve catalysts D1-D7 obtained in Comparative Examples 1-7 respectively, to evaluate the catalytic performance thereof. The results were shown in Table 3 below.

**Table 3 catalytic performance of the catalysts of Comparative Examples in a liquid phase continuous epoxidation of propylene**

| | catalyst | The conversion of hydrogen peroxide^{a}, % | The propotion of residual hydrogen peroxide^{a}, % | The selectivity for propylene oxide, % | The ratio of the main products to the by-products | The period for keeping stability of the catalyst, hour |
|---|---|---|---|---|---|---|
| C.W.E. 8 | D1 | 98.2 | 1.8 | 97.5 | 39.2 | 188 |
| C.W.E. 9 | D2 | 98.9 | 1.1 | 99.3 | 141.8 | 823 |
| C.W.E. 10 | D3 | 98.7 | 1.3 | 99.2 | 123.8 | 654 |
| C.W.E. 11 | D4 | 98.6 | 1.4 | 99.1 | 111.3 | 558 |
| C.W.E. 12 | D5 | 99.1 | 0.9 | 99.3 | 142.1 | 985 |
| C.W.E. 13 | D6 | 98.6 | 1.4 | 99.2 | 124.1 | 596 |
| C.W.E. 14 | D7 | 5.2 | 94.8 | 92.4 | 12.2 | 0 |

| | | | | | | |
|---|---|---|---|---|---|---|
| Note: a. A sample of reaction liquid was obtained when the reaction liquid was just emerged from the outlet of the reaction tube to calculate the conversion of hydrogen peroxide and the propotion of residual rate of hydrogen peroxide | | | | | | |

The embodiments of the present invention have been described in detail above. However, the present invention is not limited thereto. Various simple modifications may be made to the embodiments of the present invention within the technical scope of the present invention, including the combinations of various technical features in any other suitable way. Those simple modifications and combinations should also be regarded as the contents disclosed herein and being within the protection scope of the present disclosure.

## Claims

1. A Ti-MWW molecular sieve catalyst, wherein an X-ray photoelectron energy spectrum of the catalyst involves peaks at 458.9±0.2 eV and 464.8±0.2 eV, preferably at 458.9±0.1 eV and 464.8±0.1 eV;
preferably, the X-ray photoelectron energy spectrum of the catalyst involves peaks at 458.9±0.2 eV, 460.3±0.2 eV, 464.8±0.2 eV and 465.9±0.2 eV, preferably at 458.9±0.1 eV, 460.3±0.1 eV, 464.8±0.1 eV and 465.9±0.1 eV.

2. The catalyst according to claim 1, **characterized in that**, a UV Raman spectrum of the catalyst involves peaks at 343±4 cm⁻¹, 484±4 cm⁻¹, 699±4 cm⁻¹ and 1097±4 cm⁻¹,
preferably, intensity of the peak at 699±4 cm⁻¹ is 0.5-10 times, preferably 2-10 times that of the peak at 343±4 cm⁻¹, and intensity of the peak at 1097±4 cm⁻¹ is 0.5-10 times, preferably 2-10 times that of the peak at 343±4 cm⁻¹.

3. The catalyst according to claim 1, **characterized in that**, the catalyst has a molar ratio of silicon to titanium of 10-200, preferably 25-100; the catalyst further comprises at least one element of boron and aluminum, preferably boron; wherein the catalyst has a molar ratio of boron to silicon of 0-0.1, preferably 0-0.03, more preferably 0.005-0.03, and a molar ratio of aluminum to silicon of 0-0.1, preferably 0-0.05.

4. The catalyst according to claim 1, **characterized in that**, the catalyst has a volume of micropores of 0.03-0.15 cm³/g, preferably 0.03-0.12 cm³/g, more preferably 0.05-0.10 cm³/g; and a propotion of the volume of micropores to the total volume of pores of 1-7.5%, preferably 1-6%, more preferably 1.7-5%.

5. The catalyst according to claim 1, **characterized in that**, the catalyst is in a fully crystalline structure; preferably, the catalyst has a mechanical strength of 30-90 N/cm, preferably 40-80 N/cm.

6. A method for preparing a Ti-MWW molecular sieve catalyst, comprising steps of:
(1) subjecting a Ti-MWW molecular sieve powder, a binder, a pore-forming agent and a fluoride to shaping and calcining, to obtain a shaped product;
(2) crystallizing the shaped product of step (1) in the presence of an organic amine solution, to obtain a catalyst precursor A;
(3) treating the catalyst precursor A of step (2) with an acid solution, and calcining, to obtain a catalyst precursor B;
(4) treating the catalyst precursor B of step (3) with an organic amine solution, to obtain the catalyst.

7. The method according to claim 6, **characterized in that**, the binder comprises a silicon source and at least one selected from the group consisting of a boron source and an aluminum source; on oxides basis, the silicon source, the boron source and the aluminum source are in a molar ratio of 1:x:y, where x=0-0.5, y=0-0.5, and x+y=0.02-1.

8. The method according to claim 7, **characterized in that**, the silicon source is at least one selected from the group consisting of silica sol, sodium silicate, white carbon black and ethyl orthosilicate; the boron source is at least one selected from the group consisting of boric acid, boron oxide and borates; the aluminum source is at least one selected from the group consisting of aluminum oxide, aluminum hydroxide, sodium metaaluminate, aluminum nitrate and aluminum sulfate.

9. The method according to any one of claims 6-8, **characterized in that**, the pore-forming agent is at least one selected from the group consisting of sesbania powder, cellulose, chitosan, lignin, starch, polyethylene glycol, a triblock copolymer of poly(ethylene oxide)-poly(propylene oxide)-poly(ethylene oxide) (P123) and a copolymer of poly(ethylene oxide)-poly(propylene oxide) (F127), and the fluoride is at least one selected from the group consisting of sodium fluoride, potassium fluoride and ammonium fluoride; wherein the Ti-MWW molecular sieve powder, the binder, the pore-forming agent and the fluoride are used in a mass ratio of 1:(0.1-1.5):(0.01-0.1):(0.01-0.4).

10. The method according to claim 6, **characterized in that**, step (2) comprises a step of: placing the shaped product of step (1) over the organic amine solution but not in contact with the organic amine solution, wherein the organic amine is at least one selected from the group consisting of piperidine and hexamethyleneimine, the organic amine solution has a concentration of 0.3-15 mol/L, the shaped product and organic amine solution are used in a mass ratio of (0.1-10):1, and the crystallizing is operated at a temperature of 130-190 °C for 1-9 days.

11. The method according to claim 6, **characterized in that**, step (3) comprises a step of: subjecting the catalyst precursor A of step (2) and the acid solution to contacting and reacting, wherein the acid solution is at least one selected from the group consisting of solutions of nitric acid, hydrochloric acid, sulfuric acid, formic acid, acetic acid and oxalic acid, the acid solution has a concentration of 0.3-12 mol/L, the catalyst precursor A and the acid solution are used in a mass ratio of 1:(10-80), and the treating with the acid solution is operated at a temperature of 60-130°C for 4-48 hours.

12. The method according to claim 6, **characterized in that**, step (4) comprises a step of: subjecting the catalyst precursor B of step (3) and the organic amine solution to contacting and reacting in the presence of the fluoride, wherein the fluoride is at least one selected from the group consisting of sodium fluoride, potassium fluoride and ammonium fluoride, the organic amine is at least one selected from the group consisting of piperidine and hexamethyleneimine, the organic amine solution has a concentration of 0.3-15 mol/L, the catalyst precursor B, the fluoride and the organic amine solution are in a mass ratio of 1:(0.05-0.4):(2-20), and the treating with the organic amine solution is operated at a temperature of 130-190°C for 4-48 hours.

13. The method according to claim 6, **characterized in that**, in step (1), the calcining is operated at 450-650 °C under an oxygen-containing atmosphere for 4-12 hours; and in step (3), the calcining is operated at 450-650 °C under an oxygen-containing atmosphere for 4-12 hours.

14. The catalyst prepared by the method according to any one of claims 5-13.

15. Use of the catalyst according to any one of claims 1-5 or the catalyst according to claim 14 in epoxidation of olefins.
